# EUROPÄISCHE PATENTSCHRIFT

(11) **EP 0 545 303 B1**
(45) Veröffentlichungstag und Bekanntmachung des Hinweises auf die Patenterteilung: **01.10.1997**
(21) Anmeldenummer: 92120288.3
(22) Anmeldetag: 27.11.1992
(51) Int. Cl.: C08F 10/00, C08F 4/602

(54) **Verfahren zur Herstellung eines hochmolekularen Olefinpolymers**
Process for preparing high molecular weight polyolefins
Procédé de préparation d'une polyoléfine à haut poids moléculaire

(30) Priorität: 30.11.1991 DE 4139596
(43) Veröffentlichungstag der Anmeldung: 09.06.1993
(60) Teilanmeldung: 97101673.8
(73) Patentinhaber: HOECHST AKTIENGESELLSCHAFT, 65926 Frankfurt am Main (DE)
(72) Erfinder: Winter, Andreas, Dr., W-6246 Glashütten/Ts. (DE); Rohrmann, Jürgen, Dr., W-6233 Kelkheim/Ts. (DE); Dolle, Volker, Dr., W-6233 Kelkheim/Ts. (DE); Küber, Frank, Dr., W-6370 Oberursel (DE)

(56) Entgegenhaltungen:
- EP-A- 0 363 029
- EP-A- 0 523 416

## Beschreibung

Die Erfindung bezieht sich auf ein Verfahren zur Herstellung von Olefinpolymeren mit hoher Isotaktizität, enger Molmassenverteilung und hoher Molmasse.

Polyolefine mit hoher Molmasse besitzen insbesondere Bedeutung für die Herstellung von Folien, Platten oder Großhohlkörpern wie z.B. Rohre oder Formteile.

Aus der Literatur ist die Herstellung von Polyolefinen mit löslichen Metallocenverbindungen in Kombination mit Aluminoxanen oder anderen Cokatalysatoren, die aufgrund ihrer Lewis-Acidität das neutrale Metallocen in ein Kation überführen und stabilisieren können, bekannt.

Beispielsweise wurde eine spezielle Voraktivierungsmethode des Metallocens mit einem Aluminoxan vorgeschlagen, welche zu einer beachtlichen Steigerung der Aktivität des Katalystorsystems und zu einer deutlichen Verbesserung der Kornmorphologie des Polymeren führt (vgl. DE 37 26 067). Die Voraktivierung erhöht zwar die Molmasse, jedoch ist keine wesentliche Steigerung erreichbar.

Eine weitere, aber noch nicht ausreichende, Steigerung der Molmasse konnte durch Verwendung speziell heteroatomverbrückter Metallocene bei hoher Metallocenaktivität realisiert werden (EP-A 0 336 128).

Weiterhin sind Katalysatoren auf Basis Ethylenbisindenylhafniumdichlorid und Ethylen-bis(4,5,6,7-tetrahydro-1-indenyl)hafniumdichlorid und Methylaluminoxan bekannt, mit denen durch Suspensionspolymerisation höhermolekulare Polypropylene hergestellt werden können (vgl. J.A. Ewen et al., J. Am.Chem.Soc. 109 (1987) 6544). Unter technisch relevanten Polymerisationsbedingungen ist jedoch die Kornmorphologie der derart erzeugten Polymeren nicht befriedigend und die Aktivität der eingesetzten Katalysatoren vergleichsweise gering. Verbunden mit den hohen Katalysatorkosten ist somit mit diesen Systemen eine kostengünstige Polymerisation nicht möglich.

Eine deutliche Steigerung der Molmasse konnte durch die Verwendung von Metallocenen erreicht werden, bei denen die durch eine Brücke fixierten aromatischen π-Liganden in 2-Stellung (DE-P 40 35 886.0) oder in 2- und 4-Stellung (DE-P 41 28 238.8) Substituenten tragen.

Unter dem Zwang großtechnisch kostengünstiger Produktion muß bei möglichst hohen Reaktionstemperaturen polymerisiert werden, da bei höheren Polymerisationstemperaturen die entstehende Polymerisationswärme mit weniger Kühlmedium abgeführt werden kann und daher mit einer deutlich geringeren Dimensionierung des Kühlwasserkreislaufes realisierbar ist.

Die letztgenannten Metallocene mit Substituenten in 2- bzw. 2- und 4-Stellung zur Brücke sind in dieser Hinsicht bei 70°C Polymerisationstemperatur bereits sehr leistungsfähig, trotzdem sind die erzielbaren Molmassen bei technisch relevanten Polymerisationstemperaturen (z.B. 70°C) für einige technische Anwendungen wie beispielsweise die Herstellung von Polymeren für Rohre und Großhohlkörper sowie spezielle Fasern noch zu gering.

Es bestand die Aufgabe, ein Verfahren bzw. ein Katalysatorsystem zu finden, das Polymere mit guter Kornmorphologie und hoher Molmasse in großer Ausbeute erzeugt. Durch Verwendung von Wasserstoff als Molmassenregler kann dann die gesamte Molmassenpalette mit nur einem Metallocen abgedeckt werden.

Es wurde gefunden, daß unter Verwendung von in der Ligandsphäre in bestimmter Weise substituierten, verbrückten Metallocensystemen diese Aufgabe gelöst werden kann.

Die Erfindung betrifft somit ein Verfahren zur Herstellung eines Olefinpolymers durch Polymerisation oder Copolymerisation eines Olefins der Formel R^{a}-CH = CH-R^{b}, worin R^{a} und R^{b} gleich oder verschieden sind und ein Wasserstoffatom oder einen Kohlenwasserstoffrest mit 1 bis 14 C-Atomen bedeuten, oder R^{a} und R^{b} mit den sie verbindenden Atomen einen Ring bilden können, bei einer Temperatur von -60 bis 200°C, bei einem Druck von 0,5 bis 100 bar, in Lösung, in Suspension oder in der Gasphase, in Gegenwart eines Katalysators, welcher aus einem Metallocen als Übergangsmetallverbindung und einem Cokatalysator gebildet wird, dadurch gekennzeichnet, daß das Metallocen eine Verbindung der Formel I ist, 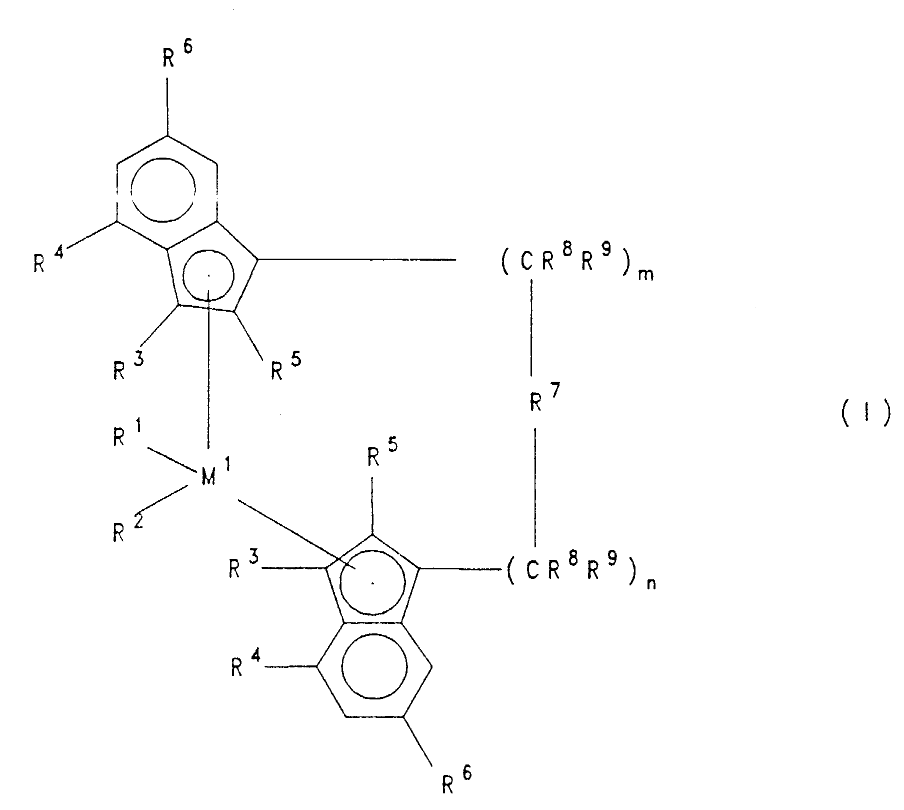 worin
- M¹: ein Metall der Gruppe IVb, Vb oder VIb des Periodensystems ist,
- R¹ und R²: gleich oder verschieden sind und ein Wasserstoffatom, eine C₁-C₁₀-Alkylgruppe, eine C₁-C₁₀-Alkoxygruppe, eine C₆-C₁₀-Arylgruppe, eine C₆-C₁₀-Aryloxygruppe, eine C₂-C₁₀-Alkenylgruppe, eine C₇-C₄₀-Arylalkylgruppe, eine C₇-C₄₀-Alkylarylgruppe, eine C₈-C₄₀-Arylalkenylgruppe oder ein Halogenatom bedeuten,
- die Reste R³: gleich oder verschieden sind und ein Wasserstoffatom, ein Halogenatom, eine C₁-C₁₀-Alkylgruppe, die halogeniert sein kann, eine C₆-C₁₀-Arylgruppe, die halogeniert sein kann, einen -NR₂¹⁰, -SR¹⁰, -OSiR₃¹⁰, -SiR₃¹⁰ oder -PR₂¹⁰-Rest bedeuten, worin R¹⁰ ein Halogenatom, eine C₁-C₁₀-Alkylgruppe oder eine C₆-C₁₀-Arylgruppe ist,
- R⁴ bis R⁶: gleich oder verschieden sind und die für R³ genannte Bedeutung haben, mit der Maßgabe, daß R⁴ und R⁶ nicht Wasserstoff sind,
- R⁷: 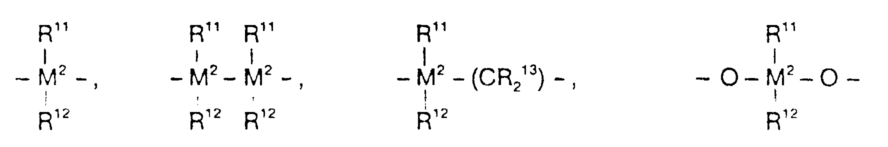 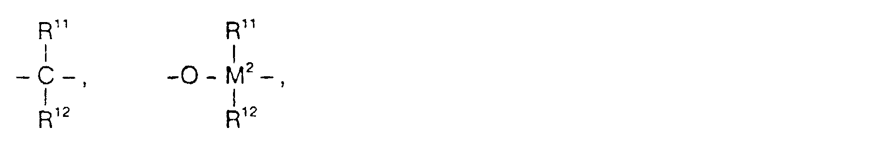
=BR¹¹, =AlR¹¹, -Ge-, -Sn-, -O-, -S-, =SO, =SO₂, =NR¹¹, =CO,
=PR¹¹ oder P(O)R¹¹ ist,
wobei
- R¹¹, R¹² und R¹³: gleich oder verschieden sind und ein Wasserstoffatom, ein Halogenatom, eine C₁-C₁₀-Alkylgruppe, C₁-C₁₀-Fluoralkylgruppe, eine C₆-C₁₀-Arylgruppe, eine C₆-C₁₀-Fluorarylgruppe, eine C₁-C₁₀-Alkoxygruppe, eine C₂-C₁₀-Alkenylgruppe, eine C₇-C₄₀-Arylalkylgruppe, eine C₈-C₄₀-Arylalkenylgruppe, eine C₇-C₄₀-Alkylarylgruppe bedeuten oder R¹¹ und R¹² oder R¹¹ und R¹³ jeweils mit den sie verbindenden Atomen einen Ring bilden,
- M²: Silizium, Germanium oder Zinn ist,
- R⁸ und R⁹: gleich oder verschieden sind und die für R¹¹ genannte Bedeutung haben und
- m und n: gleich oder verschieden sind und null, 1 oder 2 sind, wobei m plus n null, 1 oder 2 ist.

Alkyl steht für geradkettiges oder verzweigtes Alkyl. Halogen (halogeniert) bedeutet Fluor, Chlor, Brom oder Jod, bevorzugt Fluor oder Chlor.

Die Substituenten R⁴ - R⁶ an den beiden Indenylresten können trotz gleicher Bezeichnung verschieden sein (vgl. Definition von R³).

Gegenstand der vorliegenden Erfindung sind ferner die nach dem beschriebenen Verfahren hergestellten Polyolefine.

Der für das erfindungsgemäße Verfahren zu verwendende Katalysator besteht aus einem Cokatalysator und einem Metallocen der Formel I.

In Formel I ist M¹ ein Metall der Gruppe IVb, Vb oder VIb des Periodensystems, beispielsweise Titan, Zirkon, Hafnium, Vanadium, Niob, Tantal, Chrom, Molybdän, Wolfram, vorzugsweise Zirkon, Hafnium und Titan.

R¹ und R² sind gleich oder verschieden und bedeuten ein Wasserstoffatom, eine C₁-C₁₀-, vorzugsweise C₁-C₃-Alkylgruppe, eine C₁-C₁₀-, vorzugsweise C₁-C₃-Alkoxygruppe, eine C₆-C₁₀-, vorzugsweise C₆-C₈-Arylgruppe, eine C₆-C₁₀-, vorzugsweise C₆-C₈-Aryloxygruppe, eine C₂-C₁₀-, vorzugsweise C₂-C₄-Alkenylgruppe, eine C₇-C₄₀-, vorzugsweise C₇-C₁₀-Arylalkylgruppe, eine C₇-C₄₀-, vorzugsweise C₇-C₁₂-Alkylarylgruppe, eine C₈-C₄₀-, vorzugsweise C₈-C₁₂-Arylalkenylgruppe oder ein Halogenatom, vorzugsweise Chlor.

Die Reste R³ sind gleich oder verschieden und bedeuten ein Wasserstoffatom, ein Halogenatom, bevorzugt ein Fluor-, Chlor- oder Bromatom, eine C₁-C₁₀-, vorzugsweise C₁-C₄-Alkylgruppe, die halogeniert sein kann, eine C₆-C₁₀-, vorzugsweise C₆-C₉-Arylgruppe, die halogeniert sein einen -NR₂¹⁰, -SR¹⁰, -OSiR₃¹⁰, -SiR₃¹⁰ oder -PR₂¹⁰-Rest, worin R¹⁰ ein Halogenatom, vorzugsweise Chloratom, oder eine C₁-C₁₀-, vorzugsweise C₁-C₃-Alkylgruppe oder C₆-C₁₀-, vorzugsweise C₆-C₈-Arylgruppe ist. Besonders bevorzugt ist R³ Wasserstoff, C₁-C₄-Alkyl oder C₆-C₉-Aryl.

R⁴ bis R⁶ sind gleich oder verschieden und haben die für R³ beschriebene Bedeutung, mit der Maßgabe, daß R⁴ und R⁶ nicht Wasserstoff sein dürfen. Bevorzugt sind R⁴ bis R⁶ (C₁-C₄)-Alkyl oder C₆-C₉-Aryl, die beide halogeniert sein können, wie Methyl, Ethyl, Propyl, Isopropyl, Butyl, Isobutyl, Trifluormethyl, Phenyl, Tolyl oder Mesityl, insbesondere Methyl, Isopropyl oder Phenyl.

R⁷ ist 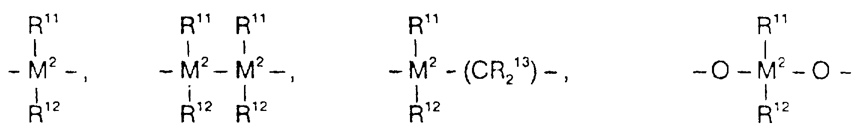 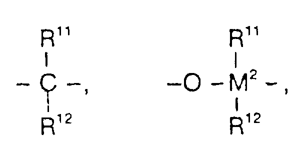 =BR¹¹, =AIR¹¹, -Ge-, -Sn-, -O-, -S-, =SO, =SO₂, =NR¹¹, =CO,
=PR¹¹ oder =P(O)R¹¹, wobei R¹¹, R¹² und R¹³ gleich oder verschieden sind und ein Wasserstoffatom, ein Halogenatom, eine C₁-C₁₀-, vorzugsweise C₁-C₄-Alkylgruppe, insbesondere Methylgruppe, eine C₁-C₁₀-Fluoralkylgruppe, vorzugsweise CF₃-Gruppe, eine C₆-C₁₀-, vorzugsweise C₆-C₈-Arylgruppe, eine C₆-C₁₀-Fluorarylgruppe, vorzugsweise Pentafluorphenylgruppe, eine C₁-C₁₀-, vorzugsweise C₁-C₄-Alkoxygruppe, insbesondere Methoxygruppe, eine C₂-C₁₀-, vorzugsweise C₂-C₄-Alkenylgruppe, eine C₇-C₄₀-, vorzugsweise C₇-C₁₀-Arylalkylgruppe, eine C₈-C₄₀-, vorzugsweise C₈-C₁₂-Arylalkenylgruppe oder eine C₇-C₄₀-, vorzugsweise C₇-C₁₂-Alkylarylgruppe bedeuten, oder R¹¹ und R¹² oder R¹¹ und R¹³ bilden jeweils zusammen mit den sie verbindenden Atomen einen Ring.

M² ist Silizium, Germanium oder Zinn, bevorzugt Silizium und Germanium.

R⁷ ist vorzugsweise =CR¹¹R¹², =SiR¹¹R¹², =GeR¹¹R¹², -O-, -S-, =SO, =PR¹¹ oder =P(O)R¹¹.

R⁸ und R⁹ sind gleich oder verschieden und haben die für R¹¹ genannte Bedeutung.

m und n sind gleich oder verschieden und bedeuten null, 1 oder 2, bevorzugt null oder 1, wobei m plus n null, 1 oder 2, bevorzugt null oder 1 ist.

Somit sind die besonders bevorzugten Metallocene die Verbindungen der Formeln A, B und C 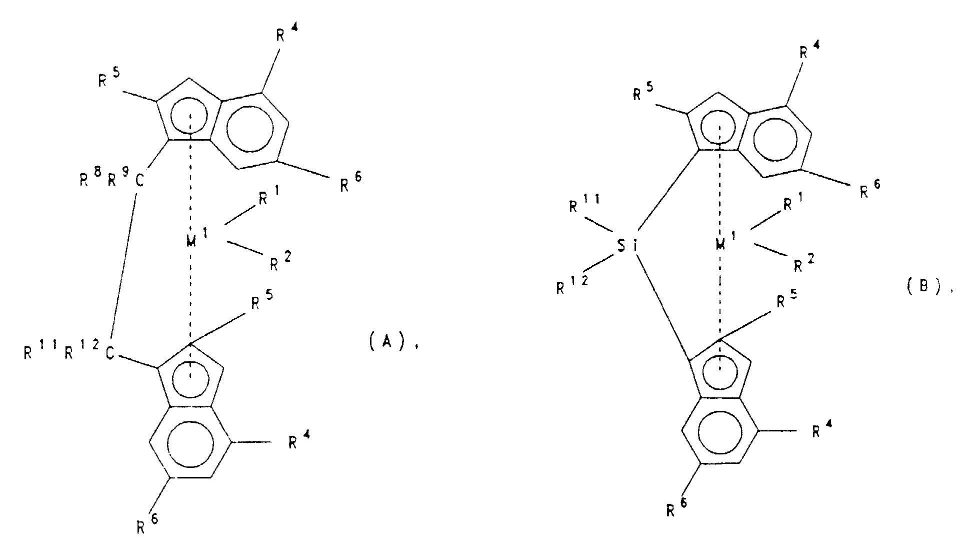 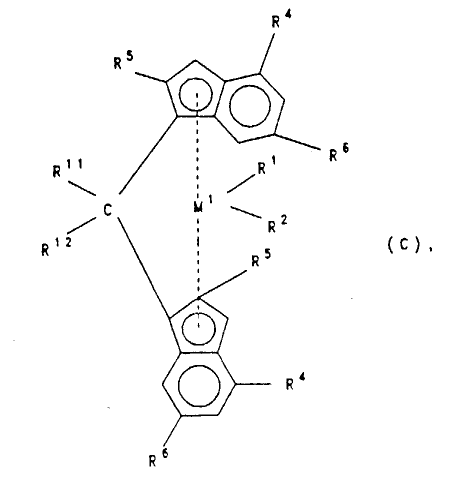 mit
M¹ = Zr, Hf; R¹, R² = Methyl, Chlor, R⁴ und R⁶ = Methyl, Isopropyl, Phenyl, Ethyl, Trifluormethyl; R⁵ = Wasserstoff und den für R⁴ und R⁶ genannten Bedeutungen,
und R⁸, R⁹, R¹¹ und R¹² mit den obengenannten Bedeutungen, insbesondere die in den Ausführungsbeispielen aufgeführten Verbindungen I.
Vorzugsweise ist R⁵ ungleich Wasserstoff und hat die Bedeutungen von R⁴ und R⁶.

Das bedeutet, daß die Indenylreste der Verbindungen I insbesondere in 2,4,6-Stellung substituiert sind (R³ = H).

Die chiralen Metallocene werden bevorzugt als Racemat eingesetzt. Verwendet werden kann aber auch die reine R- oder S-Form. Mit diesen reinen stereoisomeren Formen ist optisch aktives Polymeres herstellbar. Abgetrennt werden sollte jedoch die meso-Form der Metallocene, da das polymerisationsaktive Zentrum (das Metallatom) in diesen Verbindungen wegen Spiegelsymmetrie am Zentralmetall nicht mehr chiral ist und daher kein hochisotaktisches Polymeres erzeugen kann. Wird die meso-Form nicht abgetrennt, entsteht neben isotaktischen Polymeren auch ataktisches Polymer. Für bestimmte Anwendungen - weiche Formkörper beispielsweise - kann dies durchaus wünschenswert sein.

Die Trennung der Stereoisomeren ist im Prinzip bekannt.

Die vorstehend beschriebenen Metallocene können nach folgendem Reaktionsschema hergestellt werden:

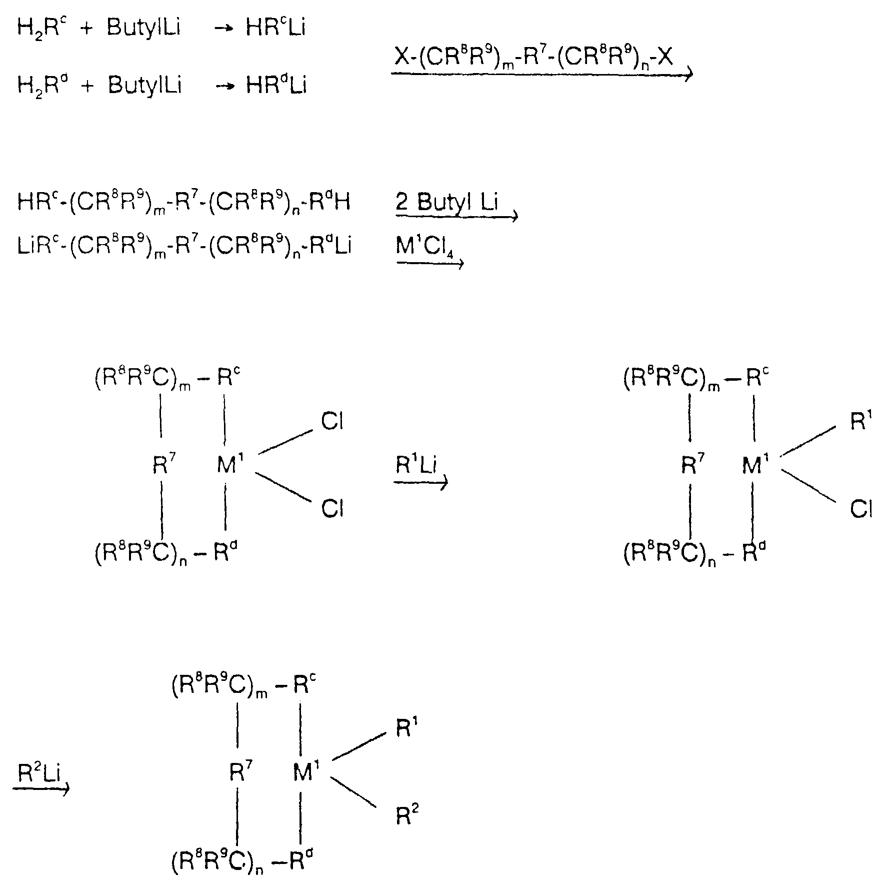

X = Cl, Br, I, O-Tosyl;

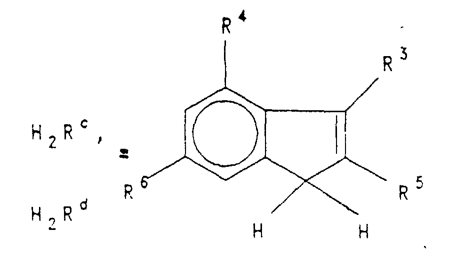

Die Herstellungsverfahren sind ausgehend von H₂R^{c}/H₂R^{d} literaturbekannt; vgl. Journal of Organometallic Chem. 288 (1985) 63-67, EP-A 320 762 und die Ausführungsbeispiele.

Die Herstellung der Verbindungen H₂R^{c}, H₂R^{d} erfolgt durch Umsetzung einer Verbindung IV 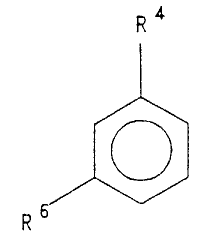 mit einer Verbindung V 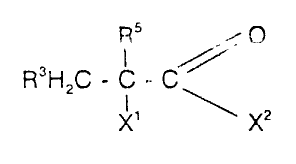 oder deren Anhydrid in Gegenwart eines Friedel-Crafts-Katalysators. Dabei stehen X¹ und X² für eine nucleophile Abgangsgruppe wie z.B. Halogen, Hydroxygruppe oder eine Tosylgruppe; insbesondere für Brom oder Chlor.

Man erhält die Indanone VI bzw. VIa

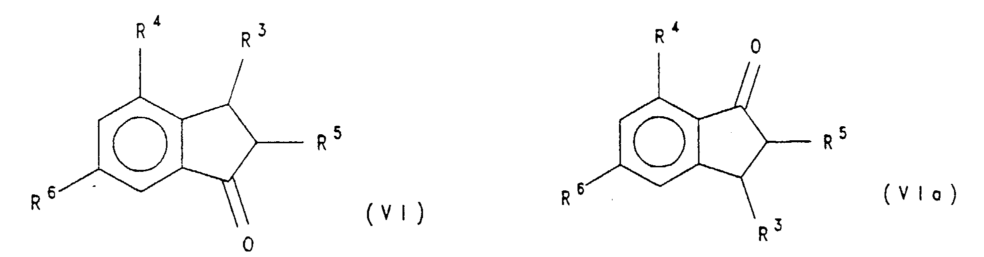

Die Indanone können in Abhängigkeit des Substitutionsmusters am Aromaten in Form zweier Konstitutionsisomere der Formel VI bzw. VIa anfallen. Diese können in reiner Form oder als Gemisch nach literaturbekannten Methoden mit Reduktionsmitteln wie NaBH₄ oder LiAIH₄ zu den entsprechenden Indanolen reduziert und anschließend mit Säuren wie Schwefelsäure, Oxalsäure, p-Toluolsulfonsäure oder auch durch Behandlung mit wasserentziehenden Substanzen wie Magnesiumsulfat, Natriumsulfat, Aluminiumoxid, Kieselgel oder Molekularsiebe zu Indenen der Formel VII bzw. VIIa (H₂R^{c}/H₂R^{d}) dehydratisiert werden (Bull. Soc. Chim. Fr. 11(1973) 3092; Organomet. 9 (1990) 3098 und die Ausführungsbeispiele). 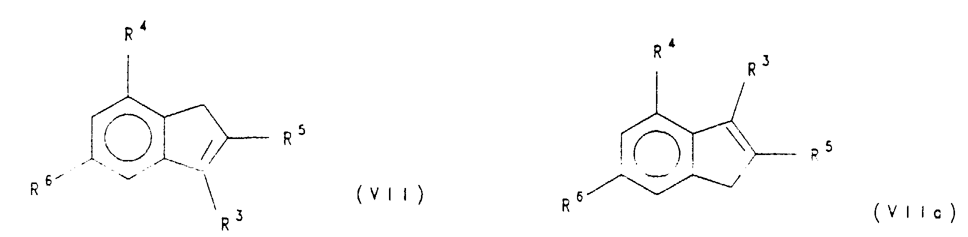 Geeignete Friedel-Crafts-Katalysatoren sind z.B. AlCl₃, AlBr₃, FeCl₃, SbCl₅, SnCl₄, BF₃, TiCl₄, ZnCl₂, HF, H₂SO₄, Polyphosphorsäure, H₃PO₄ oder eine AlCl₃/NaCl-Schmelze; insbesondere AlCl₃.

Die Ausgangsverbindungen der Formeln IV und V sind bekannt und im Handel erhältlich, oder sie lassen sich nach literaturbekannten Verfahren herstellen.

Die Umsetzung wird in einem inerten Lösemittel durchgeführt. Bevorzugt wird Methylenchlorid oder CS₂ eingesetzt. Sind die Ausgangskomponenten flüssig, kann auch auf ein Lösemittel verzichtet werden.

Die Molverhältnisse der Ausgangsverbindungen einschließlich des Friedel-Crafts-Katalysators können innerhalb weiter Grenzen schwanken. Bevorzugt ist das Molverhältnis von Verbindung I:II:Katalysator = 1:0,5-1,5:1-5; insbesondere 1:1:2,5-3.

Die Reaktionstemperatur beträgt bevorzugt 0°C bis 130°C, insbesondere 25°C bis 80°C.

Die Reaktionszeiten schwanken in der Regel zwischen 30 min und 100 h, vorzugsweise zwischen 2 h und 30 h.

Bevorzugt wird eine Mischung der Verbindungen IV und V vorgelegt und der Friedel-Crafts-Katalysator zudosiert. Auch die umgekehrte Zugabefolge ist möglich.

Die Indanone der Formel VI bzw. VIa können durch Destillation, Säulenchromatographie oder Kristallisation gereinigt werden.

Die substituierten Indene können als Doppelbindungsisomere anfallen (VII/VIIa). Diese können von Nebenprodukten durch Destillation, Säulenchromatographie oder Kristallisation gereinigt werden.

Ausgehend von den Indenen der Formeln VII und VIIa, die als Isomerengemisch eingesetzt werden können, verläuft die Herstellung der Metallocene I nach literaturbekannten Verfahren (vgl. AU-A-31478/89, J. Organomet. Chem. 342 (1988) 21, EP-A 284 707 und die Ausführungsbeispiele) entsprechend dem angegebenen Reaktionsschema.

Erfindungsgemäß wird als Cokatalysator bevorzugt ein Aluminoxan der Formel (II) 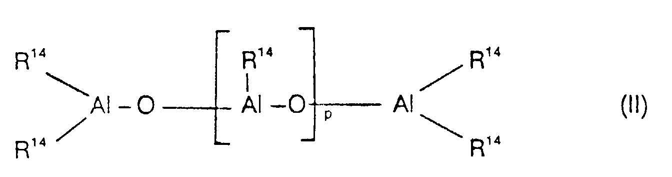 für den linearen Typ und/oder der Formel (III) 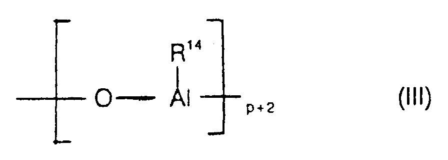 für den cyclischen Typ verwendet, wobei in den Formeln (II) und (III) die Reste R¹⁴ gleich oder verschieden sein können und eine C₁-C₆-Alkylgruppe, eine C₆-C₁₈-Arylgruppe, Benzyl oder Wasserstoff bedeuten, und p eine ganze Zahl von 2 bis 50, bevorzugt 10 bis 35 bedeutet.

Bevorzugt sind die Reste R¹⁴ gleich und bedeuten Methyl, Isobutyl, Phenyl oder Benzyl, besonders bevorzugt Methyl.

Sind die Reste R¹⁴ unterschiedlich, so sind sie bevorzugt Methyl und Wasserstoff oder alternativ Methyl und Isobutyl, wobei Wasserstoff bzw. Isobutyl bevorzugt zu 0,01 - 40 % (Zahl der Reste R¹⁴) enthalten sind.

Das Aluminoxan kann auf verschiedene Arten nach bekannten Verfahren hergestellt werden. Eine der Methoden ist beispielsweise, daß eine Aluminiumkohlenwasserstoffverbindung und/oder eine Hydridoaluminiumkohlenwasserstoffverbindung mit Wasser (gasförmig, fest, flüssig oder gebunden - beispielsweise als Kristallwasser) in einem inerten Lösungsmittel (wie z.B. Toluol) umgesetzt wird. Zur Herstellung eines Aluminoxans mit veschiedenen Alkylgruppen R¹⁴ werden entsprechend der gewünschten Zusammensetzung zwei verschiedene Aluminiumtrialkyle (AlR₃ + AlR'₃) mit Wasser umgesetzt (vgl. S. Pasynkiewicz, Polyhedron 9 (1990) 429 und EP-A 302 424).

Die genaue Struktur der Aluminoxane II und III ist nicht bekannt.

Unabhängig von der Art der Herstellung ist allen Aluminoxanlösungen ein wechselnder Gehalt an nicht umgesetzter Aluminiumausgangsverbindung, die in freier Form oder als Addukt vorliegt, gemeinsam.

Es ist möglich, das Metallocen vor dem Einsatz in der Polymerisationsreaktion mit einem Aluminoxan der Formel (II) und/oder (III) vorzuaktivieren. Dadurch wird die Polymerisationsaktivität deutlich erhöht und die Kornmorphologie verbessert.

Die Voraktivierung der Übergangsmetallverbindung wird in Lösung vorgenommen. Bevorzugt wird dabei das Metallocen in einer Lösung des Aluminoxans in einem inerten Kohlenwasserstoff aufgelöst. Als inerter Kohlenwasserstoff eignet sich ein aliphatischer oder aromatischer Kohlenwasserstoff. Bevorzugt wird Toluol verwendet.

Die Konzentration des Aluminoxans in der Lösung liegt im Bereich von ca. 1 Gew.-% bis zur Sättigungsgrenze, vorzugsweise von 5 bis 30 Gew.-%, jeweils bezogen auf die Gesamtlösung. Das Metallocen kann in der gleichen Konzentration eingesetzt werden, vorzugsweise wird es jedoch in einer Menge von 10⁻⁴ - 1 mol pro mol Aluminoxan eingesetzt. Die Voraktivierungszeit beträgt 5 Minuten bis 60 Stunden, vorzugsweise 5 bis 60 Minuten. Man arbeitet bei einer Temperatur von -78°C bis 100°C, vorzugsweise 0 bis 70°C.

Das Metallocen kann auch vorpolymerisiert oder auf einen Träger aufgebracht werden. Zur Vorpolymerisation wird bevorzugt das (oder eines der) in der Polymerisation eingesetzte(n) Olefin(e) verwendet.

Geeignete Träger sind beispielsweise Silikagele, Aluminiumoxide, festes Aluminoxan oder andere anorganische Trägermaterialien. Ein geeignetes Trägermaterial ist auch ein Polyolefinpulver in feinverteilter Form.

Erfindungsgemäß können an Stelle oder neben eines Aluminoxans Verbindungen der Formeln RₓNH₄₋ₓBR'₄, RₓPH₄₋ₓBR'₄, R₃CBR'₄ oder BR'₃ als geeignete Cokatalysatoren verwendet werden. In diesen Formeln bedeutet x eine Zahl von 1 bis 4, bevorzugt 3, die Reste R sind gleich oder verschieden, bevorzugt gleich, und bedeuten C₁-C₁₀-Alkyl, C₆-C₁₈-Aryl oder 2 Reste R bilden zusammen mit dem sie verbindenden Atom einen Ring, und die Reste R' sind gleich oder verschieden, bevorzugt gleich, und stehen für C₆-C₁₈-Aryl, das durch Alkyl, Haloalkyl oder Fluor substituiert sein kann.

Insbesondere steht R für Ethyl, Propyl, Butyl oder Phenyl und R' für Phenyl, Pentafluorphenyl, 3,5-Bistrifluormethylphenyl, Mesityl, Xylyl oder Tolyl (vgl. EP-A 277 003, EP-A 277 004 und EP-A 426 638).

Bei Verwendung der obengenannten Cokatalysatoren besteht der eigentliche (aktive) Polymerisationskatalysator aus dem Reaktionsprodukt von Metallocen und einer der genannten Verbindungen. Daher wird zunächst dieses Reaktionsprodukt bevorzugt außerhalb des Polymerisationsreaktors in einem separaten Schritt unter Verwendung eines geeigneten Lösungsmittels hergestellt.

Prinzipiell ist als Cokatalysator erfindungsgemäß jede Verbindung geeignet, die aufgrund ihrer Lewis-Acidität das neutrale Metallocen in ein Kation überführen und dieses stabilisieren kann ("labile Koordination"). Darüberhinaus soll der Cokatalysator bzw. das aus ihm gebildete Anion keine weiteren Reaktionen mit dem gebildeten Metallocenkation eingehen (vgl. EP-A 427 697).

Zur Entfernung von im Olefin vorhandener Katalystorgifte ist eine Reinigung mit einem Aluminiumalkyl, beispielsweise AlMe₃ oder AlEt₃ vorteilhaft. Diese Reinigung kann sowohl im Polymerisationssystem selbst erfolgen, oder das Olefin wird vor der Zugabe in das Polymerisationssystem mit der Al-Verbindung in Kontakt gebracht und anschließend wieder abgetrennt.

Die Polymerisation oder Copolymerisation wird in bekannter Weise in Lösung, in Suspension oder in der Gasphase, kontinuierlich oder diskontinuierlich, ein- oder mehrstufig bei einer Temperatur von -60 bis 200°C, vorzugsweise 30 bis 80°C, besonders bevorzugt 50 bis 80°C, durchgeführt. Polymerisiert oder copolymerisiert werden Olefine der Formel R^{a}-CH=CH-R^{b}. In dieser Formel sind R^{a} und R^{b} gleich oder verschieden und bedeuten ein Wasserstoffatom oder einen Alkylrest mit 1 bis 14 C-Atomen. R^{a} und R^{b} können jedoch auch mit den sie verbindenden C-Atomen einen Ring bilden. Beispiele für solche Olefine sind Ethylen, Propylen, 1-Buten, 1-Hexen, 4-Methyl-1-penten, 1-Octen, Norbornen oder Norbonadien. Insbesondere werden Propylen und Ethylen polymerisiert.

Als Molmassenregler und/oder zur Aktivitätserhöhung wird, falls erforderlich, Wasserstoff zugegeben. Der Gesamtdruck im Polymerisationssystem beträgt 0,5 bis 100 bar. Bevorzugt ist die Polymerisation in dem technisch besonders interessanten Druckbereich von 5 bis 64 bar.

Dabei wird das Metallocen in einer Konzentration, bezogen auf das Übergangsmetall, von 10⁻³ bis 10⁻⁸, vorzugsweise 10⁻⁴ bis 10⁻⁷ mol Übergangsmetall pro dm³ Lösemittel bzw. pro dm³ Reaktorvolumen angewendet. Das Aluminoxan wird in einer Konzentration von 10⁻⁵ bis 10⁻¹ mol, vorzugsweise 10⁻⁴ bis 10⁻² mol pro dm³ Lösemittel bzw. pro dm³ Reaktorvolumen verwendet. Die anderen genannten Cokatalysatoren werden in etwa äquimolaren Mengen zum Metallocen verwendet. Prinzipiell sind aber auch höhere Konzentrationen möglich.

Wenn die Polymerisation als Suspensions- oder Lösungspolymerisation durchgeführt wird, wird ein für das Ziegler- Niederdruckverfahren gebräuchliches inertes Lösemittel verwendet. Beispielsweise arbeitet man in einem aliphatischen oder cycloaliphatischen Kohlenwasserstoff; als solcher sei beispielsweise Propan, Butan, Pentan, Hexan, Heptan, Isooctan, Cyclohexan, Methylcyclohexan, genannt.

Weiterhin kann eine Benzin- bzw. hydrierte Dieselölfraktion benutzt werden. Brauchbar ist auch Toluol. Bevorzugt wird im flüssigen Monomeren polymerisiert.

Werden inerte Lösemittel verwendet, werden die Monomeren gasförmig oder flüssig zudosiert.

Die Dauer der Polymerisation ist beliebig, da das erfindungsgemäß zu verwendende Katalysatorsystem einen nur geringen zeitabhängigen Abfall der Polymerisationsaktivität zeigt.

Das erfindungsgemäße Verfahren zeichnet sich dadurch aus, daß die beschriebenen Metallocene im technisch besonders interessanten Temperaturbereich zwischen 50 und 80°C Polymere mit hoher Molmasse, hoher Stereospezifität und guter Kornmorphologie erzeugen.

Insbesondere die erfindungsgemäßen Zirkonocene stoßen in einen Molmassenbereich vor, oder übertreffen diesen sogar, der beim bisherigen Stand der Technik den Hafnocenen vorbehalten war. Diese hatten jedoch den Nachteil nur geringer Polymerisationsaktivität und sehr hoher Katalysatorkosten und die damit hergestellten Polymeren wiesen eine schlechte Pulvermorphologie auf.

Die nachfolgenden Beispiele sollen die Erfindung näher erläutern.

Es bedeuten:
- VZ =: Viskositätszahl in cm³/g 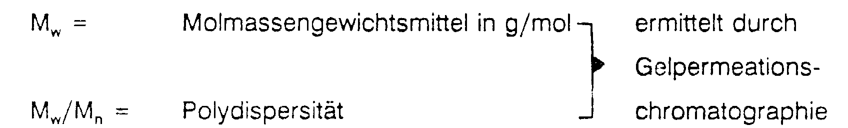
- Schmp. =: Schmelzpunkt ermittelt mit DSC (20°C/min Aufheiz-/Abkühlgeschwindigkeit)
- II =: Isotaktischer Index (II = mm+1/2 mr) ermittelt durch ¹³C-NMR-Spektroskopie
- MFI/(230/5): Schmelzindex, gemessen nach DIN 53735; in dg/min
- SD =: Polymerschüttdichte in g/dm³

Synthese der in den Beispielen verwendeten Metallocene:

### Beispiel A

### 2,5,7-Trimethyl-1-indanon (1)

Eine Lösung von 34,4 g (324 mmol) m-Xylol (99 %) und 74 g (324 mmol) 2-Bromisobuttersäurebromid (98 %) in 600 ml Methylenchlorid p. a. wurde bei Raumtemperatur unter kräftigem Rühren über einen Feststoffdosiertrichter langsam mit 107 g (810 mmol) AlCl₃ versetzt, wobei eine heftige Gasentwicklung einsetzte. Die Mischung wurde 15 h bei Raumtemperatur gerührt, auf Eiswasser gegossen, das mit 25 ml konz. HCl angesäuert wurde und mehrmals ausgeethert. Die vereinigten organischen Phasen wurden zunächst mit einer gesättigten NaHCO₃₋ Lösung und dann mit einer gesättigten NaCl-Lösung gewaschen und mit Magnesiumsulfat getrocknet. Das nach Abziehen des Lösemittels unter vermindertem Druck zurückgebliebene Öl wurde über eine kurze Destillationsbrücke destilliert. Bei 81-90°C/0,1-0,2 mbar gingen 52,4 g des Indanons 1 in Form eines farblosen Öls über, das bei Raumtemperatur kristallisierte. Die Ausbeute betrug 93 %.
¹H-NMR-Spektrum (100 MHz, CDCl₃): 7,05 (1,s), 6,87 (1,s), 3,25 (1,q), 2,43-2,80 (2,m), 2,57 (3,s), 2,35 (3,s), 1,25 (3,d).
Massenspektrum: 174 M⁺, korrektes Zerfallsmuster.

### Beispiel B

### 2,4,6-Trimethylinden (2)

20,4 g (117 mmol) 2,5,7-Trimethyl-1-indanon (1) wurden in 300 ml einer Mischung aus Tetrahydrofuran/Methanol (2:1) gelöst und bei Raumtemperatur mit 6,6 g (175 mmol) NaBH₄ versetzt. Die Mischung wurde noch 1 h gerührt, mit 50 ml halbkonz. HCl versetzt und ausgeethert. Die vereinigten organischen Phasen wurden über Natriumsulfat getrocknet und vom Lösemittel befreit. Der Rückstand wurde in eine Destillationsapparatur überführt und mit 13 g Magnesiumsulfat versetzt. Es wurde ein Vakuum von ca. 10 mbar angelgt und hochgeheizt, bis das Produkt überdestillierte (130-150°C). Die Destillation ergab 17,7 g des Indens 2 als farbloses Öl. Die Ausbeute betrug 96 %.
Massenspektrum: 158 M⁺, korrektes Zerfallsmuster.

### Beispiel C

### 2-Methyl-5,7-diisopropyl-1-indanon (3) bzw. 2-Methyl-4,6-diisopropyl-1-indanon (3a)

Eine Lösung von 84,8 g (523 mmol) 1,3-Diisopropylbenzol und 120 g (523 mmol) 2-Bromisobuttersäurebromid (98 %) in 600 ml Methylenchlorid p. a. wurde bei Raumtemperatur über einen Feststoffdosiertrichter langsam mit 174 g (1300 mmol) AlCl₃ versetzt. Die Mischung wurde noch 20 h zum Rückfluß erhitzt und dann analog Beispiel A aufgearbeitet. Das Rohprodukt wurde an 3 kg Kieselgel 60 chromatographiert. Die Indanone 3 und 3a konnten mit einem Laufmittelgemisch aus Hexan/15 % Essigester separat eluiert werden. Mit dem gleichen Laufmittel folgte in einer weiteren Zone als Nebenprodukt die Verbindung 2-Methyl-5-isopropyl-1-indanon. Eine Trennung der beiden Isomere ist für die weiteren Umsetzungen jedoch nicht erforderlich. Die Gesamtausbeute betrug 93 g (78 %). ¹H-NMR-Spektrum (360 MHz, CDCl₃): Isomerenmischung (3:2) 7,49 (d), 7,36 (d). 7,13 (s), 7,10 (s), 4,15 (sept.), 3,25-3,40 (m), 3,10 (sept.), 2,90-3,00 (m), 2,60-2,73 (m), 1,22-1,30 (m).
Massenspektrum: 230 M⁺, korrektes Zerfallsmuster.

### Beispiel D

### 2-Methyl-4,6-diisopropylinden (4) bzw. 2-Methyl-5,7-diisopropylinden (4a), Variante I

Eine Lösung von 78,5 g (341 mmol) der Isomerenmischung 3/3a in 700 ml eines Lösemittelgemisches aus Tetrahydrofuran/Methanol p.a. (2:1) wurde bei Raumtemperatur mit 19,3 g (511 mmol) NaBH₄ versetzt. Nach 2 h Rühren bei Raumtemperatur wurden 120-130 ml halbkonz. HCl zugesetzt und ausgeethert. Die vereinigten organischen Phasen wurden mit Na₂SO₄ getrocknet. Der nach Abziehen des Lösemittels verbliebene Rückstand wurde in 500 ml Methylenchlorid aufgenommen und mit 6,5 g (34 mmol) p-Toluolsulfonsäure 15 min zum Rückfluß erhitzt. Der nach Abziehen des Lösemittels verbliebene Rückstand wurde an 1,5 kg Kieselgel 60 chromatographiert. Mit einem Laufmittelgemisch aus Hexan/Diisopropylether 20:1 ließen sich 56 g der Isomerenmischung 4/4a in Form eines farblosen Öls isolieren. Die Gesamtausbeute betrug 86 %. ¹H-NMR-Spektrum (100 MHz, CDCl₃): Doppelbindungsisomere (1:1) 7,1 (m), 6,95 (m), 6,60 (m), 6,43 (m), 3,25 (br), 2,75-3,20 (m), 2,12 (d), 1,28 (d), 1,25 (d). Massenspektrum: 214 M⁺, korrektes Zerfallsmuster.

### Beispiel E

### 2-Methyl-4,6-diisopropylinden (4) bzw. 2-Methyl-5,7-diisopropylinden (4a), Variante II

Eine Lösung von 78,5 g (341 mmol) der Isomerenmischung 3/3a in 700 ml eines Lösemittelgemisches aus Tetrahydrofuran/Methanol p.a. (2:1) wurde bei Raumtemperatur mit 19,3 g (511 mmol) NaBH₄ versetzt. Nach 2 h Rühren bei Raumtemperatur wurden 120-130 ml halbkonz. HCl zugesetzt und ausgeethert. Die vereinigten organischen Phasen wurden mit Na₂SO₄ getrocknet. Der nach Abziehen des Lösemittels verbliebene Rückstand wurde in eine Destillationsapparatur überführt und mit 50 g Magnesiumsulfat versetzt. Nach Anlegen eines Vakuums von ca. 1 mbar wurde hochgeheizt, bis das Produkt überging (ca. 130°C). Man erhielt 65 g der Isomerenmischung 4/4a als farbloses Öl. Die Ausbeute betrug 90 %.

### Beispiel F

### Dimethylbis(2-Methyl-4,6-diisopropylindenyl)silan (5)

Eine Lösung von 4,9 g (22,8 mmol) der Isomerenmischung 4/4a in 25 ml THF wurde bei 0°C unter Ar-Schutz mit 9,2 ml (22,8 mmol) einer 2,5 M Butyllithiumlösung in Hexan versetzt und noch 1 h zum Rückfluß erhitzt. Die rote Lösung wurde anschließend bei Raumtemperatur zu einer Lösung von 1,5 g (11,4 ml) Dimethyldichlorsilan in 10 ml THF getropft und 8 h unter Rückfluß erhitzt. Der Ansatz wurde auf Eiswasser gegossen und ausgeethert. Die über Magnesiumsulfat getrocknete Etherphase wurde unter vermindertem Druck eingedampft. Das zurückgebliebene gelbliche Öl wurde anschließend an 500 g Kieselgel 60 chromatographiert. Mit einem Laufmittelgemisch aus Hexan/5 % Methylenchlorid konnten zunächst 1,4 g der Indenmischung 4/4a eluiert werden. Mit Hexan/8 % Methylenchlorid folgte das Ligandsystem 11. Das nach Abziehen des Laufmittels verbliebene viskose Öl konnte durch Verrühren mit Methanol im Eisbad kristallisiert werden. Es wurden 3,1 g eines gelblichen Feststoffs erhalten. Die Ausbeute betrug 56 % bzw. 84 % bezüglich umgesetztem Inden.
¹H-NMR-Spektrum (100 MHz, CDCl₃): Doppelbindungsisomere (3:1) 6,82-7,32 (m), 6,70 (m), 6,62 (m), 6,52 (m), 3,75 (s,br), 3,65 (s,br), 3,35 (s), 2,70-3,30 (m), 2,05-2,25 (m), 1,10-1,45 (m), 0,10-0,22 (m), -0,15 bis -0,32 (m).
Massenspektrum: 484 M⁺, korrekter Zerfall.

### Beispiel G

### Dimethylsilandiylbis(2-Methyl-4,6-diisopropylindenyl)zirkondichlorid (6)

Eine Lösung von 3,1 g (6,5 mmol) des Ligandsystems 5 in 25 ml Diethylether wurde bei Raumtemperatur unter Ar-Schutz mit 6,3 ml (16,2 mmol) einer 2,5 M Butyllithiumlösung in Hexan versetzt und über Nacht gerührt. Nach Zusatz von 10 ml Hexan wurde die beigefarbene Suspension filtriert und der Rückstand mit 20 ml Hexan gewaschen. Das Dilithiosalz wurde lange im Ölpumpenvakuum getrocknet und dann bei -78°C zu einer Suspension von 1,6 g (6,8 mmol) ZrCl₄ in 30 ml Methylenchlorid gegeben. Die Mischung wurde innerhalb 1 h auf Raumtemperatur erwärmt und noch 30 min bei dieser Temperatur gerührt. Nach dem Abziehen des Lösemittels wurde der orangebraune Rückstand mit 50 ml Hexan extrahiert. Nach Abziehen des Lösemittels erhielt man 2,6 g (60 %) des Komplexes 12 in Form eines gelben Pulvers. Das Verhältnis des Racemats zur meso-Form war 3:1. Durch Umkristallisation aus Hexan konnten 1,3 g (30 %) des Komplexes 12 als reines Racemat gewonnen werden (gelbes Kristallpulver). ¹H-NMR-Spektrum (100 MHz, CDCl₃): 7,27 (2,s,Arom.-H), 7,05 (2,s,Arom.-H), 6,80 (2,s,ß-Ind-H), 2,6-3,2 (4,m,i-Pr-CH), 2,22 (6,s,Ind-CH₃), 1,15-1,40 (30,m,i-Pr-CH₃, Si-CH₃). Massenspektrum: 642 M⁺ (bzgl.⁹⁰Zr), korrektes Isotopenmuster, korrekter Zerfall.

### Beispiel H

### Dimethylbis(2,4,6-trimethylindenyl)silan (7)

Eine Lösung von 10,1 g (64 mmol) des Indens 2 in 50 ml THF wurde bei Raumtemperatur unter Ar-Schutz mit 25,5 ml (63,7 mmol) einer 2,5 M Butyllithiumlösung in Hexan versetzt und 1 h zum Rückfluß erhitzt. Die so erhaltene Lösung wurde bei Raumtemperatur zu einer Lösung von 4,1 g (32 mmol) Dimethyldichlorsilan in 20 ml THF getropft und 3 h zum Rückfluß erhitzt. Die Reaktionsmischung wurde auf Eiswasser gegossen und mehrmals ausgeethert. Die vereinigten organischen Phasen wurden über Natriumsulfat getrocknet und unter vermindertem Druck eingedampft. Der Rückstand wurde an 450 g Kieselgel 60 chromatographiert. Mit einem Laufmittelgemisch aus Hexan/5 % Methylenchlorid ließen sich zunächst 2,5 g des Indens 2 eluieren. Mit Hexan/8 % Methylenchlorid folgten 6,5 g des Ligandsystems 7 (Isomere). Die Ausbeute betrug 54 % bzw. 72 % bezüglich umgesetztem Inden 2.

### Beispiel J

### Dimethylsilandiylbis(2,4,6-trimethylindenyl)zirkondichlorid (8)

Eine Lösung von 2,0 g (5,4 mmol) des Ligandsystems 7 in 30 ml Diethylether wurde bei Raumtemperatur unter Ar-Schutz mit 6,6 ml (16,2 mmol) einer 2,5 M Butyllithiumlösung in Hexan versetzt und 5-6 h bei dieser Temperatur gerührt. Die Lösung wurde vollständig eingedampft. Der verbleibende feste Rückstand wurde portionsweise mit insgesamt 30 ml Hexan gewaschen und lange im Ölpumpenvakuum getrocknet. Das so erhaltene beigefarbene Pulver wurde bei - 78°C zu einer Suspension von 1,23 g (5,5 mmol) Zirkontetrachlorid in 30 ml Methylenchlorid gegeben. Nach dem Aufwärmen auf Raumtemperatur wurde die Reaktionsmischung vollständig eingedampft und im Ölpumpenvakuum getrocknet. Der feste Rückstand bestand aus einer Mischung der racemischen mit der meso-Form im Verhältnis 1:1. Dieser wurde zunächst mit einer kleinen Menge Hexan gewaschen. Anschließend wurde mit insgesamt 120 ml Toluol extrahiert. Die Lösung wurde eingeengt und bei -35°C der Kristallisation überlassen. Es ließen sich 800 mg (28 %) des Zirkonocens 8 als reines Racemat in Form orangefarbener Kristalle gewinnen.
¹H-NMR-Spektrum des Racemats (100 MHz, CDCl₃): 7,20 (s,2,Arom.-H), 6,97 (s,2,Arom.-H), 6,70 (s,2,ß-Ind-H), 2,32 (s,6,CH₃), 2,27 (s,6,CH₃), 2,20 (s,6,CH₃), 1,27 (s,6,Si-CH₃).
Massenspektrum: 530 M⁺ (bzgl. ⁹⁰Zr), korrektes Isotopenmuster, korrekter Zerfall.

### Beispiel K

### Methylphenylbis(2-methyl-4,6-diisopropylindenyl)silan (9).

Eine Lösung von 10 g (46 mmol) des Indens 4/4a in 200 ml THF wurde unter Ar-Schutz bei Raumtemperatuzr mit 18,6 ml (46 mmol) einer 2,5 M Butyllithiumlösung in Hexan versetzt und 1 h zum Rückfluß erhitzt. Die Lösung wurde bei Raumtemperatur zu einer Lösung von 4,48 g (23 mmol) Methylphenyldichlorsilan in 30 ml THF getropft und 3 h zum Rückfluß erhitzt. Die Mischung wurde auf Eiswasser gegossen und mehrmals ausgeethert. Die vereinigten organischen Phasen wurden mit Natriumsulfat getrocknet und eingedampft. Der Rückstand wurde an 450 g Kieselgel 60 chromatographiert. Mit einem Laufmittelgemisch aus Hexan/Methylenchlorid (10:1) konnten zunächst 1,9 g nicht umgesetztes Inden 4/4a zurückgewonnen werden. Anschließend folgten 7,4 g des Ligandsystems 9 (Isomerengemsich). Die Ausbeute betrug 57 % bzw. 73 % bezüglich umgesetztem Inden.

### Beispiel L

### Methylphenylsilylbis(2-methyl-4,6-diisopropylindenyl)zirkondichlorid (10)

Eine Lösung von 7,4 g (13,5 mmol) des Ligandsystems 9 in 30 ml Diethylether wurde bei Raumtemperatur unter Ar-Schutz mit 11,2 ml (28 mmol) einer 2,5 M Butyllithiumlösung in Hexan versetzt und 16 h bei Raumtemperatur gerührt. Nach dem Abziehen des Lösemittels wurde der verbleibende Rückstand 3-4 h bei 40-50°C getrocknet und anschließend bei -78°C zu einer Suspension von 3,2 g (13,5 mmol) Zirkontetrachlorid in 40 ml Methylenchlorid gegeben. Nach dem Aufwärmen auf Raumtemperatur wurde das Lösemittel unter vermindertem Druck abgezogen. Der verbleibende feste Rückstand wurde im Ölpumpenvakuum getrocknet und mit 100 ml Hexan extrahiert. Nach Abziehen des Lösemittels erhält man 5,4 g (55 %) des Zirkonocens 10 als Mischung der racemischen mit der meso-Form im Verhältnis 2:1 (orangebraunes Kristallpulver). Durch Umkristallisation aus Hexan ist die reine racemische Form erhältlich.
¹H-NMR-Spektrum der Isomerenmischung (100 MHz, CDCl₃): 6,6-8,2 (m,Arom.-H,β-Ind-H), 2,5-3,2 (m,i-Pr-H), 2,52 (s,CH₃), 2,32 (s,CH₃), 2,20 (s,CH₃), 1,90 (s,CH₃), 1,0-1,5 (m,i-Pr-CH₃,Si-CH₃).
Massenspektrum: 704 M⁺ (bzgl. ⁹⁰Zr), korrektes Isotopenmuster, korrekter Zerfall.

### Beispiel M

### 1,2-Bis(2-methyl-4,6-diisopropylindenyl)ethyl (11)

Eine Lösung von 5,0 g (23,3 mmol) des Indens 4/4a in 50 ml THF wurde unter Ar-Schutz bei Raumtemperatur mit 18,6 ml (46 mmol) einer 2,5 M Butyllithiumlösung in Hexan versetzt und 1 h zum Rückfluß erhitzt. Die Lösung wurde bei -78°C zu einer Lösung von 2,18 g (11,0 mmol) 1,2-Dibromethan gegeben. Die Lösung wurde langsam auf Raumtemperatur erwärmt und über Nacht bei dieser Temperatur gerührt. Die Mischung wurde auf Eiswasser gegossen und mehrmals ausgeethert. Die vereinigten organischen Phasen wurden mit Natriumsulfat getrocknet und eingedampft. Der Rückstand wurde an 450 g Kieselgel 60 chromatographiert. Mit einem Laufmittelgemisch aus Hexan/Methylenchlorid (20:1 bis 10:1) konnten zunächst 1,2 g nicht umgesetztes Inden 4/4a zurückgewonnen werden. Anschließend folgten 1,7 g des Ligandsystems 11 (farbloser Feststoff). Die Ausbeute betrug 35 % bzw. 45 % bezüglich umgesetztem Inden.

### Beispiel N

### 1,2-Ethandiylbis(2-methyl-4,6-diisopropylindenyl)zirkondichlorid (12)

Eine Lösung von 1,6 g (3,52 mmol) des Ligandsystems 11 in 20 ml Diethylether wurde bei Raumtemperatur unter Ar-Schutz mit 3,5 ml (8,8 mmol) einer 2,5 M Butyllithiumlösung in Hexan versetzt und über Nacht gerührt. Der nach Abziehen des Lösemittels verbleibende Rückstand wurde mit Hexan gewaschen und lange im Ölpumpenvakuum getrocknet. Das so erhaltene Pulver wurde bei -78°C zu einer Suspension von 815 mg (3,5 mmol) Zirkontetrachlorid in 15 ml Methylenchlorid gegeben. Nach dem Erwärmen auf Raumtemperatur wurde noch 1 h gerührt und das Lösemittel unter vermindertem Druck entfernt. Der im Ölpumpenvakuum getrocknete Rückstand wurde mit Toluol extrahiert. Nach Abziehen des Lösemittels und Waschen mit Hexan erhielt man 1,5 g (70 %) des Zirkonocens 18 als Mischung der racemischen mit de meso-Form im Verhältnis 2:1 (orangefarbenes Pulver). Durch Umkristallisation aus einem Toluol/Hexan-Gemisch ließen sich 700 mg (32 %) des reinen Racemats gewinnen.
¹H-NMR-Spektrum des Racemats (100 MHz,CDCl₃): 7,3 (s,Arom.-H), 7,0 (s,Arom.-H), 6,55 (s,ß-Ind-H), 3,6 (s,C₂H₄), 2,6-3,2 (m,i-Pr-H), 2,2 (s,CH₃), 1,0-1,5 (m,i-Pr-CH₃)
Massenspektrum: 612 M⁺ (bzgl. ⁹⁰Zr), korrektes Isotopenmuster, korrekter Zerfall.

### Beispiel O

### 1,2-Bis(2-methyl4,6-diisopropylindenyl)butan (13)

Eine Lösung von 5,0 g (23,3 mmol) des Indens 4/4a wurde analog Beispiel M mit 2,37 g (11 mmol) 1,2-Dibrombutan (97 %) umgesetzt. Die Chromatographie an Kieselgel lieferte mit Hexan als Laufmittel nach Edukt und einem Nebenprodukt (Spiroverbindung) 1,16 g (22 %) des Liganden 13 als Isomerengemisch. Die Isomere konnten durch eine nochmalige Chromatographie an einer langen Säule getrennt bzw. angereichert werden.

### Beispiel P

### rac-1,2-Butandiylbis(2-methyl-4,6-diisopropylindenyl)zirkondichlorid (14)

Eine Lösung von 1,0 g (2,07 mmol) des Ligandsystems 13 (2 Isomere) in 15 ml Diethylether wurde analog Beispiel N umgesetzt. Die Umkristallisation aus Toluol/Hexan-Mischungen lieferte bei -35°C insgesamt 1,24 g (60 %) des Metallocens 14 als Mischung der verschiedenen Diastereomere der racemischen und der meso-Form. Durch nochmalige Umkristallisation konnte das Racemat 14 als 2 Diastereomere erhalten werden.
¹H-NMR-Spektrum (100 MHz, CDCl₃): 7,0-7,4 (m,Aromaten-H) 6,5 und 6,6 (s,β-Ind-H), 3,3-3,7 (m,C₂H₃), 1,0-2,8 (m,CH₃,i-Pr, C₂H₅). Massenspektrum: 640 M⁺, korrektes Isotopenmuster, korrekter Zerfall.

### Polymerisationsbeispiele:

### Beispiel I

Ein trockener 24 dm³-Reaktor wurde mit Propylen gespült und mit 12 dm³ flüssigem Propylen befüllt. Dann wurden 35 cm³ toluolische Methylaluminoxanlösung (entsprechend 52 mmol Al, mittlerer Oligomerisierungsgrad p = 20) zugegeben und der Ansatz bei 30°C 15 Minuten gerührt.

Parallel du wurden 3,5 mg (0,005 mmol) rac-Dimethylsilyl (2-methyl-4,6-diisopropyl-1-indenyl)₂ zirkondichlorid in 13,5 cm³ toluolischer Methylaluminoxanlösung (20 mmol Al) gelöst und durch 15 minütiges Stehenlassen voraktiviert.

Die weinrote Lösung wurde dann in den Reaktor gegeben, durch Wärmezufuhr auf 75°C aufgeheizt (10°C/min) und das Polymerisationssystem 1 h durch Kühlung bei 75°C gehalten. Gestoppt wurde die Polymerisation durch Abgasen des überschüssigen Monomeren. Es wurden 2,11 kg Polypropylen erhalten. Die Aktivität des Metallocens betrug somit 603 kgPP/g Metallocen x h.
VZ = 259 cm³/g, M_{w} = 305 000 g/mol; M_{w}/Mₙ = 2,0;
II = 96,0 %, MFI (230/5) = 8,5 dg/min.

### Vergleichsbeispiel 1

Beispiel 1 wurde mit dem Metallocen rac-Dimethylsilyl(2-methyl-1-indenyl)₂ zirkondichlorid wiederholt. Die Metallocenaktivität war 395 kg PP/g Metallocen x h, VZ = 159 cm³/g, M_{w} = 158 000 g/mol, M_{w}/Mₙ = 2,1 und der MFI (230/5) war 48 dg/min. Der isotaktische Index (II) betrug 96,0 %.

### Vergleichsbeispiel 2

Beispiel 1 wurde mit dem Metallocen rac-Dimethylsilyl(2-methyl-4-isopropyl-1-indenyl)₂zirkondichlorid wiederholt. Die Metallocenaktivität war 460 kg PP/g Metallocen x h, VZ = 152 cm³/g, M_{w} = 147 500 g/mol, M_{w}/Mₙ = 2,3 und MFI (230/5) = 51 dg/min.

### Vergleichsbeispiel 3

Beispiel 1 wurde mit dem Metallocen rac-Dimethylsilyl(1-indenyl)₂zirkondichlorid wiederholt. Die Metallocenaktivität war 695 kg PP/g Metallocen x h, VZ = 31 cm³/g, M_{w} = 18 500 g/mol, M_{w}/Mₙ = 2,2, MFI (230/5) war nicht mehr meßbar.

### Vergleichsbeispiel 4

Beispiel 1 wurde mit dem Metallocen rac-Dimethylsilyl(4,7-dimethyl-1-indenyl)₂ zirkondichlorid wiederholt. Die Metallocenaktivität war 195 kg PP/g Metallocen x h, VZ = 16 cm³/g, M_{w} = 9 500 g/mol, M_{w}/Mₙ = 2,0, II = 87 %, der MFI (230/5) war nicht mehr meßbar.

Die vier Vergleichsversuche zeigen, daß mit den am Indenylliganden in unterschiedlicher Weise substituierten Metallocenen bzw. mit dem unsubstituierten Metallocen hergestellte Polypropylene deutliche Molmassenunterschiede aufweisen. Unter Einbeziehung des erfindungsgemäßen Metallocens aus Beispiel 1 reicht die Spanne vom Wachsbereich (Vergleichsbeispiel 4) bis zum sehr hochmolekularen erfindungsgemäßen Polymer (Beispiel 1).
Diese Versuche belegen die Überlegenheit der in 2,4,6-Stellung substituierten Metallocene.

### Vergleichsbeispiel 5

Beispiel 1 wurde mit dem Metallocen rac-Dimethylsilyl(3-methyl-1-indenyl)₂ zirkondichlorid wiederholt.
Es wurde ein Polypropylen mit nicht akzeptablem isotaktischen Index und mit niedriger Molmasse erhalten.

### Beispiel 2

Beispiel 1 wurde mit 5,1 mg (0,008 mmol) des Metallocens rac-Dimethylsilyl(2-methyl-4,6-diisopropyl-1-indenyl)₂zirkondichlorid wiederholt. Die Polymerisationstemperatur war 50°C. Es wurden 0,85 kg Polypropylen, entsprechend einer Metallocenaktivität von 166,7 kg PP/g Metallocen x h erhalten.
VZ = 454 cm³/g, M_{w} = 498 500 g/mol, M_{w}/Mₙ = 2,2,II = 97,1 %, MFI (230/5) = 1,7 dg/min.

### Beispiel 3

Beispiel 1 wurde mit 4,5 mg (0,007 mmol) des Metallocens rac-Dimethylsilyl(2-methyl-4,6-diisopropyl-1-indenyl)₂-zirkondichlorid bei 60°C Polymerisationstemperatur wiederholt. Es wurden 1,34 kg Polypropylen, entsprechend einer Metallocenaktivität von 298 kg PP/g Metallocen x h erhalten.
VZ = 347 cm³/g; M_{w} = 444500 g/mol, M_{w}/Mₙ = 2,1; II = 97,0 %; MFI (230/5) = 3,2 dg/min, Schmp. = 145°C.

### Beispiel 4

Beispiel 1 wurde mit 9,6 mg (0,015 mmol) des Metallocens rac-Dimethylsilyl(2-methyl-4,6-diisopropyl-1-indenyl)₂-zirkondichlorid bei 30°C Polymerisationstemperatur wiederholt. Diese Polymerisationstemperatur ist zwar großtechnisch weniger geeignet, der Versuch belegt jedoch das Molmassenpotential und die hohe Aktivität des Metallocens.
Es wurden 0,61 kg Polypropylen, entsprechend einer Metallocenaktivität von 63,5 kg PP/g Metallocen x h erhalten.
VZ = 645 cm³/g; M_{w} = 867000 g/mol, M_{w}/Mₙ = 2,1; II = 97,7 %; MFI (2320/5) = 0,26 dg/min.

### Beispiel 5

Beispiel 1 wurde mit 3,3 mg (0,006 mmol) des Metallocens rac-Dimethylsilyl(2,4,6-trimethyl-1-indenyl)₂zirkondichlorid wiederholt. Es wurden 1,83 kg Polypropylen erhalten. Die Metallocenaktivität war somit 555 kg PP/g Metallocen x h.
VZ = 165 cm³/g, M_{w} = 186 000 g/mol; M_{w}/Mₙ = 2,0; Schmp. = 145°C; MFI (230/5) = 40 dg/min.

### Beispiel 6

Beispiel 1 wurde mit 4,4 mg (0,006 mmol) des Metallocens rac-Phenyl(Methyl)silyl(2-methyl-4,6-diisopropyl-1-indenyl)₂zirkondichlorid bei 70°C Polymerisationstemperatur wiederholt. Es wurden 2,05 kg Polypropylen, entsprechend einer Metallocenaktivität von 466 kg PP/g Metallocen x h erhalten.
VZ = 263 cm³/g, M_{w} = 385 500 g/mol, M_{w}/Mₙ = 2,5, Schmp. = 145°C, MFI (230/5) = 8,5 dg/min.

### Beispiel 7

Beispiel 6 wurde mit 7,9 mg (0,011 mmol) des Metallocens bei 50°C Polymerisationstemperatur wiederholt. Die Metallocenaktivität war 156 kg PP/g Metallocen x h.
VZ = 498 cm³/g, M_{w} = 586 000 g/mol, M_{w}/Mₙ = 3.0, Schmp. = 147°C, MFI (230/5) = 1,5 dg/min.

### Beispiel 8

Beispiel 6 wurde mit 12,0 mg (0,017 mmol) des Metallocens bei 30°C Polymerisationstemperatur wiederholt. Die Metallocenaktivität war 58,3 kg PP/g Metallocen x h.
VZ = 811 cm³/g, M_{w} = 1 020 000 g/mol, M_{w}/Mₙ = 2,3, Schmp. = 148°C, MFI (230/5) = 0,2 dg/min.

### Beispiel 9

Beispiel 7 wurde mit 2,8 mg des Metallocens wiederholt. In den Reaktor wurde vor der Polymerisation 24 Ndm³ Wasserstoff eindosiert. Die Metallocenaktivität war 600 kg PP/g Metallocen x h.
VZ = 30 cm³/g, M_{w} = 18 250 g/mol, M_{w}/Mₙ = 2,5, Schmp. = 144°C.

### Beispiel 10

Beispiel 7 wurde mit 3,5 mg des Metallocens und mit 60 Ndm³ Wasserstoff wiederholf. Die Metallocenaktivität war 650 kg PP/g Metallocen x h.
VZ = 14 cm³/g, M_{w} = 6300 g/mol, M_{w}/Mₙ = 2,2, Schmp. = 145°C.

Die Beispiele 9 und 10 belegen die excellente Wasserstoffansprechbarkeit des Metallocens zur Einstellung einer gewünschten Molmasse. Mit geringen Mengen Wasserstoff läßt sich die Kettenlänge in weiten Grenzen bis in den Wachsbereich variieren.

### Beispiel 11

Ein trockener 150 dm³-Reaktor wurde mit Stickstoff gespült und bei 20°C mit 80 dm³ eines entaromatisierten Benzinschnittes mit dem Siedebereich 100-120°C gefüllt. Dann wurde der Gasraum durch 5-maliges Aufdrücken von 2 bar Propylen und Entspannen stickstofftrei gespült.
Nach Zugabe von 50 l flüssigem Propylen wurden 64 cm³ toluolische Methylaluminoxanlösung (entsprechend 100 mmol Al, Molmasse nach kryoskopischer Bestimmung 1050 g (mol)) zugegeben und der Reaktorinhalt auf 50°C aufgeheizt. Durch Zudosierung von Wasserstoff wurde ein Wasserstoffgehalt im Gasraum des Reaktors von 0,2 % eingestellt und später dann durch Nachdosierung während der gesamten Polymerisationszeit konstant gehalten (Überprüfung on-Line durch Gaschromatographie).
15,5 mg rac-Phenyl(Methyl)silyl(2-methyl-4,6-diisopropyl-1-indenyl)₂zirkondichlorid wurden in 32 ml toluolischer Methylaluminoxanlösung (entsprechend 50 mmol Al) gelöst und nach 15 Minuten in den Reaktor gegeben.

Durch Kühlung wurde der Reaktor 18 h bei 50°C Polymeristionstemperatur gehalten, dann wurde durch Zugabe von 2 bar CO₂-Gas die Polymerisation gestoppt und das gebildete Polymere auf einer Druckmutsche vom Suspensionsmedium abgetrennt. Die Trocknung des Produktes erfolgte 24 h bei 80°C/200 mbar. Es wurden 20,9 kg Polymerpulver, entsprechend einer Metallocenaktivität von 74,9 kg PP/g Metallocen x h erhalten.
VZ = 424 cm³/g, M_{w} = 518 000 g/mol, M_{w}/Mₙ = 2,0, Smp. = 149°C, MFI (230/5) = 4,1 dg/min.

### Beispiel 12

Ein trockener 24 dm³-Reaktor wurde mit Propylen gespült und mit 2,4 Ndm³ Wasserstoff und 12 dm³ flüssigem Propylen befüllt. Dann wurden 35 cm³ toluolische Methylaluminoxanlösung (entsprechend 52 mmol Al, mittlerer Oligomerisierungsgrad p = 20) zugegeben.
Parallel dazu wurden 6,5 mg rac-Phenyl(Methyl)-silyl(2-methyl-4,6-diisopropyl-1-indenyl)zirkondichlorid in 13,5 cm³ toluolischer Methylaluminoxanlösung (20 mmol Al) gelöst und durch 5 minütiges Stehenlassen voraktiviert.
Die Lösung wurde dann in den Reaktor gegeben und unter kontinuierlicher Zugabe von 60 g Ethylen wurde 1 h bei 60°C polymerisiert. Die Metallocenaktivität betrug 398 kg PP/g Metallocen x h. Der Ethylengehalt des Copolymeren betrug 2,0 %.
VZ = 503 cm³/g, M_{w} = 384 000 g/mol, M_{w}/Mₙ = 2,0, Smp. = 139°C, laut NMR-Spektroskopie wurde das Ethylen überwiegend isoliert (statistisches Copolymer) eingebaut.

### Beispiel 13

Ein trockener 150 dm³-Reaktor wurde wie in Beispiel 11 befüllt.
18,9 mg rac-Phenyl(Methyl)silyl(2-methyl-4,6-diisopropyl-1-indenyl)₂zirkondichlorid wurden in 32 ml toluolischer Methylaluminoxanlösung (50 mmol) gelöst und in den Reaktor gegeben.
Die Polymerisation erfolgte in einer 1. Stufe bei 70°C 5 Stunden lang. In einer 2. Stufe wurden dann bei 55°C 3 kg Ethylen schnell zugegeben und nach weiteren 3 h Polymerisation bei 55°C wurde mit CO₂-Gas gestoppt. Es wurden 25,9 kg Blockcopolymerpulver erhalten.
VZ = 344 cm³/g, M_{w} = 399 000 g/mol, M_{w}/Mₙ = 3,8, MFI (230/5) = 5,0 dg/min.

Das Blockcopolymer enthielt 10,8 Gew.-% Ethylen. Die Fraktionierung ergab einen Gehalt von 27,5 Gew.-% Ethylen/Propylen-Kautschuk. Die Glastemperatur des Kautschuks war -51 °C.

### Beispiel 14

Beispiel 1 wurde bei 70°C Polymerisationstemperatur mit 4,0 mg des Metallocens rac-1,2-Ethandiylbis(2-methyl-4,6-diisopropyl-1-indenyl)zirkondichlorid wiederholt. Die Metallocenaktivität war 529 kg PP/g Metallocen x h.
VZ = 149 cm³/g, M_{w} = 174 500 g/mol, M_{w}/Mₙ = 1,9, Schmp. = 141 °C, MFI (230/5) = 74 dg/min.

### Beispiel 15

Beispiel 14 wurde mit 4,0 mg des Metallocens rac-1,2-Butandiylbis(2-methyl-4,6-diisopropyl-1-indenyl)zirkondichlorid wiederholt. Die Metallocenaktivität war 319 kgPP/g Metallocen x h.
VZ = 295 cm³/g, M_{w} = 368 500 g/mol, M_{w}/Mₙ = 2,1, Schmp. = 142°C, MFI (230/5) = 4,0 dg/min.

## Patentansprüche

1. Verfahren zur Herstellung eines Olefinpolymers durch Polymerisation oder Copolymerisation eines Olefins der Formel R^{a}-CH = CH-R^{b}, worin R^{a} und R^{b} gleich oder verschieden sind und ein Wasserstoffatom oder einen Kohlenwasserstoffrest mit 1 bis 14 C-Atomen bedeuten, oder R^{a} und R^{b} mit den sie verbindenden Atomen einen Ring bilden können, bei einer Temperatur von -60 bis 200°C, bei einem Druck von 0,5 bis 100 bar, in Lösung, in Suspension oder in der Gasphase, in Gegenwart eines Katalysators, welcher aus einem Metallocen als Übergangsmetallverbindung und einem Cokatalysator gebildet wird, wobei das Metallocen eine Verbindung der Formel I ist 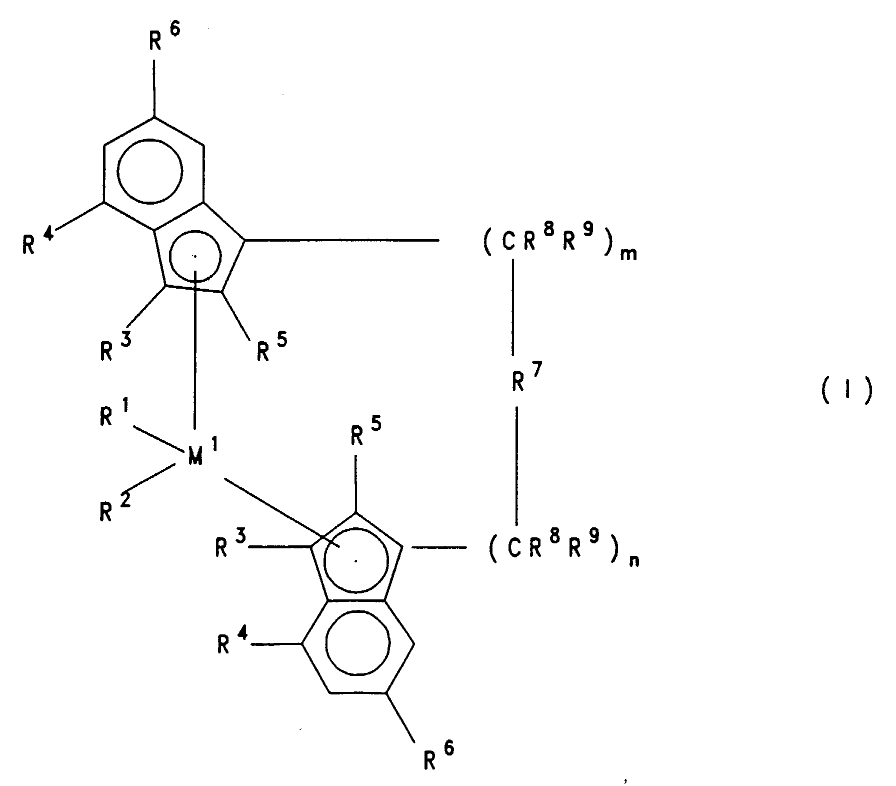 worin
M¹ ein Metall der Gruppe IVb, Vb oder Vlb des Periodensystems ist,
R¹ und R² gleich oder verschieden sind und ein Wasserstoffatom, eine C₁-C₁₀-Alkylgruppe, eine C₁-C₁₀-Alkoxygruppe, eine C₆-C₁₀-Arylgruppe, eine C₆-C₁₀-Aryloxygruppe, eine C₂-C₁₀-Alkenylgruppe, eine C₇-C₄₀-Arylalkylgruppe, eine C₇-C₄₀-Alkylarylgruppe, eine C₈-C₄₀-Arylalkenylgruppe oder ein Halogenatom bedeuten,
die Reste R³ gleich oder verschieden sind und ein Wasserstoffatom, ein Halogenatom, eine C₁-C₁₀-Alkylgruppe, die halogeniert sein kann, eine C₆-C₁₀-Arylgruppe, die halogeniert sein kann, einen - NR₂¹⁰, -SR¹⁰, -OSiR₃¹⁰, -SiR₃¹⁰ oder -PR₂¹⁰-Rest bedeuten, worin R¹⁰ ein Halogenatom, eine C₁-C₁₀-Alkylgruppe oder eine C₆-C₁₀-Arylgruppe ist,
R⁴ bis R⁶ gleich oder verschieden sind und die für R³ genannte Bedeutung haben,
R⁷ 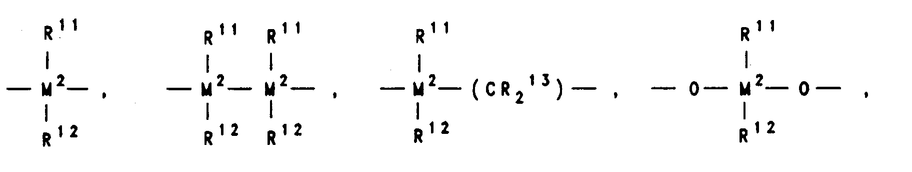 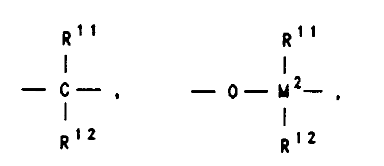
=BR¹¹, =AlR¹¹, -Ge-, -Sn-, -O-, -S-, =SO, =SO₂, =NR¹¹, =CO,
= PR¹¹ oder = P(O)R¹¹ ist,
wobei
R¹¹, R¹² und R¹³ gleich oder verschieden sind und ein Wasserstoffatom, ein Halogenatom, eine C₁-C₁₀-Alkylgruppe, C₁-C₁₀-Fluoralkylgruppe, eine C₆-C₁₀-Arylgruppe, eine C₆-C₁₀-Fluorarylgruppe, eine C₁-C₁₀-Alkoxygruppe, eine C₂-C₁₀-Alkenylgruppe, eine C₇-C₄₀-Arylalkylgruppe, eine C₈-C₄₀-Arylalkenylgruppe, eine C₇-C₄₀-Alkylarylgruppe bedeuten oder R¹¹ und R¹² oder R¹¹ und R¹³ jeweils mit den sie verbindenden Atomen einen Ring bilden,
M² Silizium, Germanium oder Zinn ist,
R⁸ und R⁹ gleich oder verschieden sind und die für R¹¹ genannte Bedeutung haben und
m und n gleich oder verschieden sind und null, 1 oder 2 sind, wobei m plus n null, 1 oder 2 ist,
dadurch gekennzeichnet, daß R⁴ und R⁶ nicht Wasserstoff sind.

2. Verfahren gemäß Anspruch 1, dadurch gekennzeichnet, daß in Formel I R³ Wasserstoff bedeutet.

3. Verfahren gemäß Anspruch 1 oder 2, dadurch gekennzeichnet, daß in Formel I M¹ Zr oder Hf. R¹ und R² gleich oder verschieden sind und Methyl oder Chlor, R⁴ und R⁶ gleich oder verschieden sind und Methyl, Isopropyl, Phenyl, Ethyl oder Trifluormethyl,R⁵ Wasserstoff ist oder die für R⁴ und R⁶ genannten Bedeutengen hat, R⁷ einen Rest 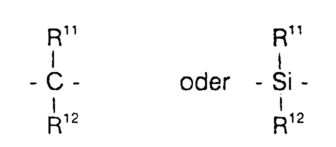 und m plus n null oder 1 bedeuten.

4. Verfahren gemäß einem oder mehreren der Ansprüche 1 bis 3, dadurch gekennzeichnet, daß als Metallocen der Formel I rac-Dimethylsilyl(2-methyl-4,6-diisopropyl-1-indenyl)₂-zirkondichlorid verwendet wird.

5. Verfahren gemäß einem oder mehreren der Ansprüche 1 bis 4, dadurch gekennzeichnet, daß als Cokatalysator ein Aluminoxan der Formel (II) 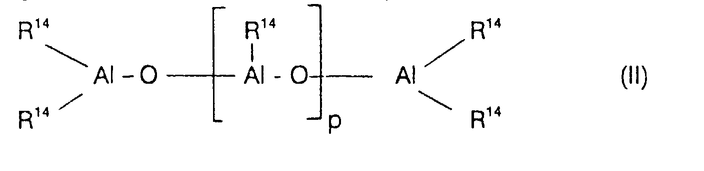 für den linearen Typ und/oder der Formel (III) 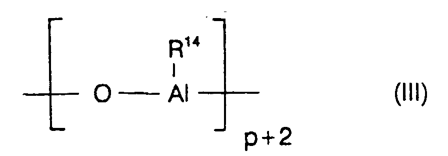 für den cyclischen Typ verwendet wird,
wobei in den Formeln (II) und (III) die Reste R¹⁴ gleich oder verschieden sind und eine C₁-C₆-Alkylgruppe, eine C₆-C₁₈-Arylgruppe, Benzyl oder Wasserstoff bedeuten und p eine ganze Zahl von 2 bis 50 ist.

6. Verfahren gemäß einem oder mehreren der Ansprüche 1 bis 5, dadurch gekennzeichnet, daß als Cokatalysator Methylaluminoxan verwendet wird.

7. Verfahren gemäß Anspruch 5 oder 6, dadurch gekennzeichnet, daß das Metallocen der Formel I vor dem Einsatz in der Polymerisationsreaktion mit einem Aluminoxan der Formel II und/oder III voraktiviert wird.

8. Verwendung eines Metallocens der Formel I gemäß einem oder mehreren der Ansprüche 1 bis 4 als Katalysator bei der Olefinpolymerisation.

9. Metallocenverbindung der Formel I 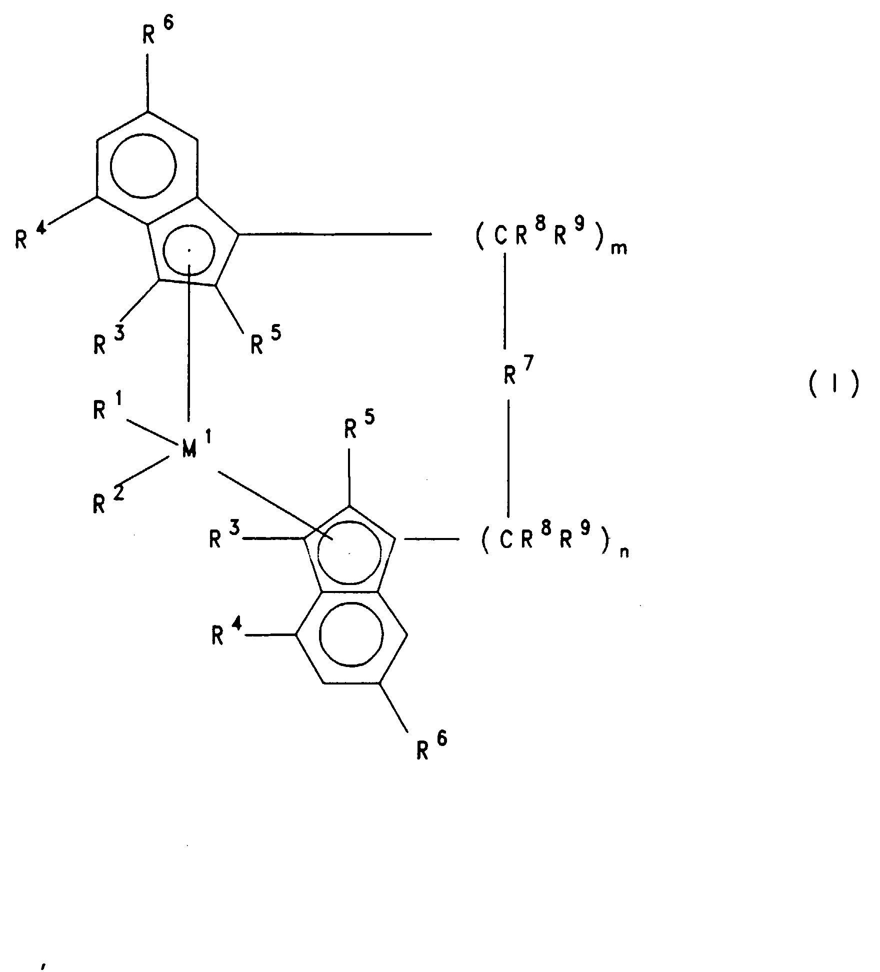 worin
M¹ ein Metall der Gruppe IVb, Vb oder Vlb des Periodensystems ist,
R¹ und R² gleich oder verschieden sind und ein Wasserstoffatom, eine C₁-C₁₀-Alkylgruppe, eine C₁-C₁₀-Alkoxygruppe, eine C₆-C₁₀-Arylgruppe, eine C₆-C₁₀-Aryloxygruppe, eine C₂-C₁₀-Alkenylgruppe, eine C₇-C₄₀-Arylalkylgruppe, eine C₇-C₄₀-Alkylarylgruppe, eine C₈-C₄₀-Arylalkenylgruppe oder ein Halogenatom bedeuten,
die Reste R³ gleich oder verschieden sind und ein Wasserstoffatom, ein Halogenatom, eine C₁-C₁₀-Alkylgruppe, die halogeniert sein kann, eine C₆-C₁₀-Arylgruppe, die halogeniert sein kann, einen - NR₂¹⁰, -SR¹⁰, -OSiR₃¹⁰, -SiR₃¹⁰ oder -PR₂¹⁰-Rest bedeuten, worin R¹⁰ ein Halogenatom, eine C₁-C₁₀-Alkylgruppe oder eine C₆-C₁₀-Arylgruppe ist,
R⁴ bis R⁶ gleich oder verschieden sind und die für R³ genannte Bedeutung haben,
R⁷ 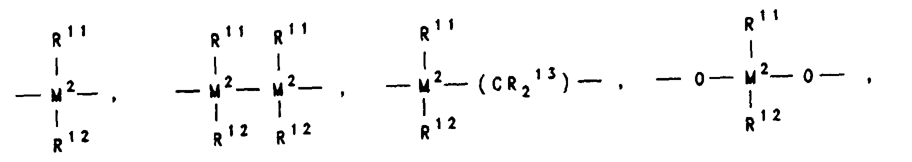 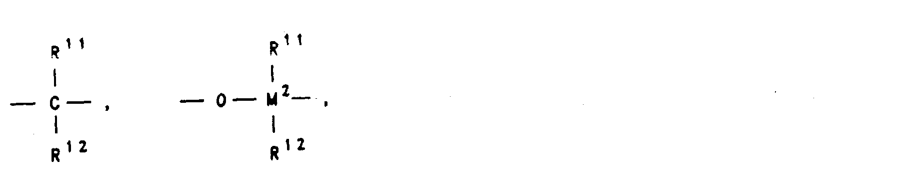
=BR¹¹, =AlR¹¹, -Ge-, -Sn-, -O-, -S-, =SO, =SO₂, =NR¹¹, =CO, =PR¹¹ oder =P(O)R¹¹ ist,
wobei
R¹¹, R¹² und R¹³ gleich oder verschieden sind und ein Wasserstoffatom, ein Halogenatom, eine C₁-C₁₀-Alkylgruppe, C₁-C₁₀-Fluoralkylgruppe, eine C₆-C₁₀-Arylgruppe, eine C₆-C₁₀-Fluorarylgruppe, eine C₁-C₁₀-Alkoxygruppe, eine C₂-C₁₀-Alkenylgruppe, eine C₇-C₄₀-Arylalkylgruppe, eine C₈-C₄₀-Arylalkenylgruppe, eine C₇-C₄₀-Alkylarylgruppe bedeuten oder R¹¹ und R¹² oder R¹¹ und R¹³ jeweils mit den sie verbindenden Atomen einen Ring bilden,
M² Silizium, Germanium oder Zinn ist,
R⁸ und R⁹ gleich oder verschieden sind und die für R¹¹ genannte Bedeutung haben und
m und n gleich oder verschieden sind und null, 1 oder 2 sind, wobei m plus n null, 1 oder 2 ist, dadurch gekennzeichnet, daß R⁴ und R⁶ nicht Wasserstoff sind.

10. Metallocen-Verbindung gemäß Anspruch 9, dadurch gekennzeichnet, daß in Formel I R³ Wasserstoff bedeutet.

11. Metallocen-Verbindung gemäß Anspruch 9 oder 10, dadurch gekennzeichnet, daß in Formel I M¹ Zr oder Hf, R¹ und R² gleich oder verschieden sind und Methyl oder Chlor, R⁴ und R⁶ gleich oder verschieden sind und Methyl, Isopropyl, Phenyl, Ethyl oder Trifluormethyl,R⁶ Wasserstoff ist oder die für R⁴ und R⁶ genannten Bedeutungen hat, R⁷ einen Rest 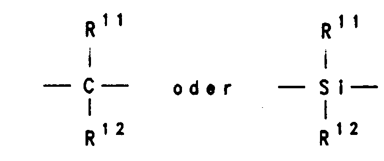 und m plus n null oder 1 bedeuten.

12. Metallocenverbing gemäß Anspruch 9 worin die Metallocenverbindung rac-Dimethylsilyl(2-methyl-4,6-diisopropyl-1-indenyl)₂ zirkondichlorid, rac-Dimethylsilyl(2,4,6-trimethyl-1-indenyl)₂ zirkondichlorid, rac-Phenyl(Methyl)silyl(2-methyl-4-6-diisopropyl-1-indenyl)₂ zirkondichlorid, rac-1,2-Ethandiylbis(2-methyl-4,6-diisopropyl-1-indenyl) zirkondichlorid oder rac-1,2-Butandiylbis(2-methyl-4,6-diisopropyl-1-indenyl) zirkondichlorid ist.

13. Katalysatorkomponente, enthaltend a) ein Metallocen gemäß einen oder mehreren der Ansprüche 9 - 12 und b) einen Cokatalysator.

## Claims

1. A process for the preparation of an olefin polymer by polymerization or copolymerization of an olefin of the formula R^{a}-CH=CH-R^{b}, in which R^{a} and R^{b} are identical or different and are a hydrogen atom or a hydrocarbon radical having I to 14 carbon atoms, or R^{a} and R^{b}, with the atoms joining them, can form a ring, at a temperature of from -60 to 200°C, under a pressure of 0.5 to 100 bar, in solution, in suspension or in the gas phase, in the presence of a catalyst which is formed from a metallocene as the transition metal compound and a cocatalyst, wherein the metallocene is a compound of the formula I 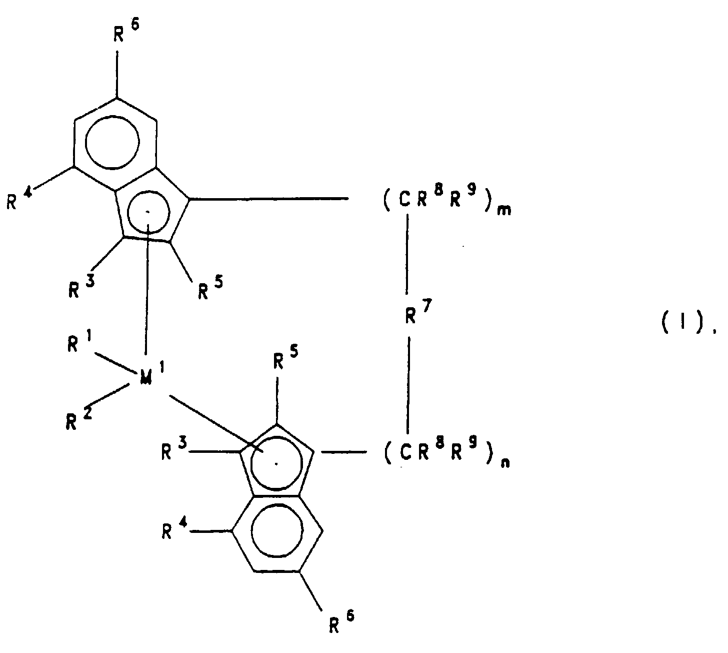 in which
M¹ is a metal of group IVb, Vb or VIb of the periodic table,
R¹ and R² are identical or different and are a hydrogen atom, a C₁-C₁₀-alkyl group, a C₁-C₁₀-alkoxy group, a C₆-C₁₀-aryl group, a C₆-C₁₀-aryloxy group, a C₂-C₁₀-alkenyl group, a C₇-C₄₀-arylalkyl group, a C₇-C₄₀-alkylaryl group, a C₈-C₄₀-arylalkenyl group or a halogen atom,
the radicals R³ are identical or different and are a hydrogen atom, a halogen atom, a C₁-C₁₀-alkyl group, which can be halogenated, a C₆-C₁₀-aryl group, which can be halogenated, or a -NR₂¹⁰, -SR¹⁰, -OSiR₃¹⁰, -SiR₃¹⁰ or -PR₂¹⁰ radical, in which R¹⁰ is a halogen atom, a C₁-C₁₀-alkyl group or a C₆-C₁₀-aryl group,
R⁴ to R⁶ are identical or different and have the meaning given for R³,
R⁷ is 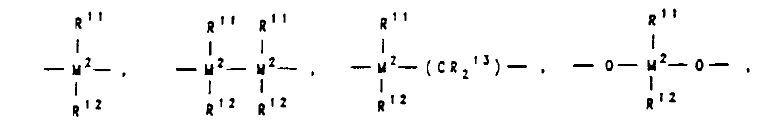 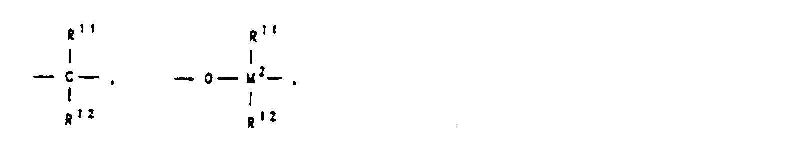
=BR¹¹, =AIR¹¹, -Ge-, -Sn-, -O-, -S-, =SO, =SO₂, =NR¹¹, =CO, -PR¹¹ or P(O)R¹¹,
in which
R¹¹, R¹² and R¹³ are identical or different and are a hydrogen atom, a halogen atom, a C₁-C₁₀-alkyl group, a C₁-C₁₀-fluoroalkyl group, a C₆-C₁₀-aryl group, a C₆-C₁₀-fluoroaryl group, a C₁-C₁₀-alkoxy group, a C₂-C₁₀-alkenyl group, a C₇-C₄₀-arylalkyl group, a C₈-C₄₀-arylalkenyl group or a C₇-C₄₀-alkyl-aryl group, or R¹¹ and R¹², or R¹¹ and R¹³, in each case with the atoms joining them, form a ring,
M² is silicon, germanium or tin,
R⁸ and R⁹ are identical or different and have the meaning given for R¹¹ and
m and n are identical or different and are zero, 1 or 2, m plus n being zero, 1 or 2,
where in which R⁴ and R⁶ are not hydrogen.

2. The process as claimed in claim 1, wherein, in formula I, R³ is hydrogen.

3. The process as claimed in claim 1 or 2, wherein, in formula I, M¹ is Zr or Hf, R¹ and R² are identical or different and are methyl or chlorine, R⁴ and R⁶ are identical or different and are methyl, isopropyl, phenyl, ethyl or trifluoromethyl, R⁵ is hydrogen or has the meanings given for R⁴ and R⁶, R⁷ is a radical 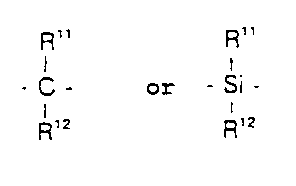 and m plus n is zero or 1.

4. The process as claimed in one or more of claims 1 to 3, wherein rac-dimethylsilyl(2-methyl-4,6-diisopropyl-1-indenyl)₂-zirconium dichloride is used as the metallocene of the formula I.

5. The process as claimed in one or more of claims 1 to 4, wherein an aluminoxane of the formula (II) 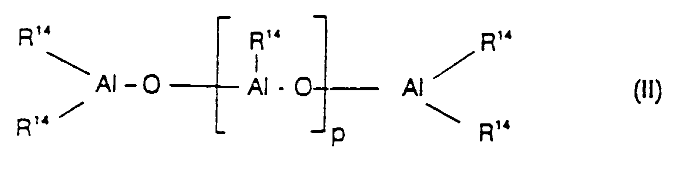 for the linear type, and/or of the formula (III) 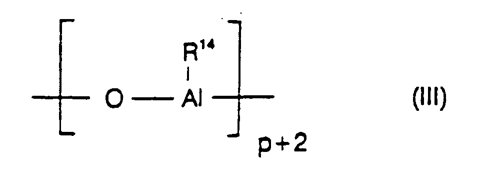 for the cyclic type,
in which, in the formulae (II) and (III), the radicals R¹⁴ are identical or different and are a C₁-C₆-alkyl group, a C₆-C₁₈-aryl group, benzyl or hydrogen and p is an integer from 2 to 50,
is used as the cocatalyst.

6. The process as claimed in one or more of claims I to 5, wherein methylaluminoxane is used as the cocatalyst.

7. The process as claimed in claim 5 or 6, wherein, before use in the polymerization reaction, the metallocene of the formula I is preactivated with an aluminoxane of the formula II and/or III.

8. The use of a metallocene of the formula I as claimed in one or more of claims 1 to 4 as a catalyst in olefin polymerization.

9. A metallocene compound of the formula I 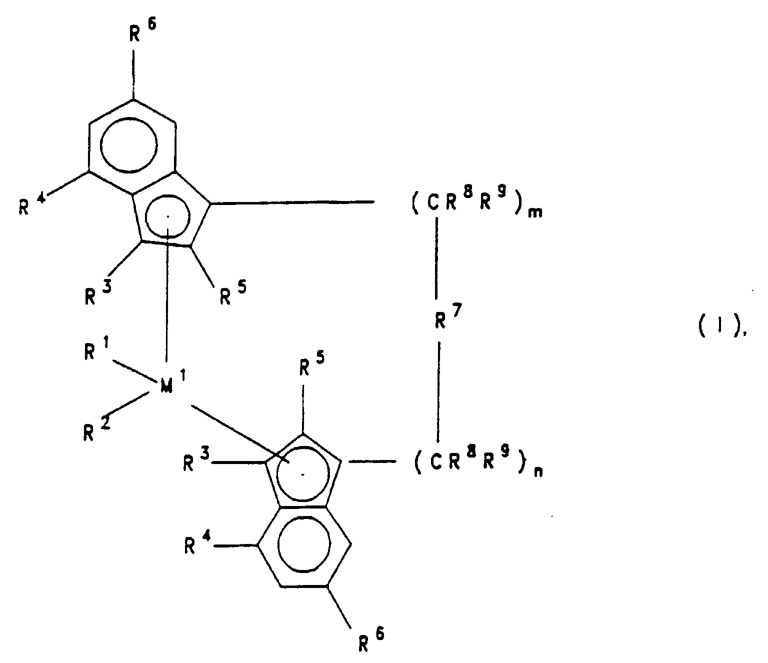 in which
M¹ is a metal of group IVb, Vb or VIb of the periodic table, C₁₀-alkoxy group, a C₆-C₁₀-aryl group, a C₆-C₁₀-aryloxy group, a C₂-C₁₀-alkenyl group, a C₇-C₄₀-arylalkyl group, a C₇-C₄₀-alkylaryl group, a C₈-C₄₀-arylalkenyl group or a halogen atom,
the radicals R³ are identical or different and are a hydrogen atom, a halogen atom, a C₁-C₁₀-alkyl group, which can be halogenated, a C₆-C₁₀-aryl group, which can be halogenated, or a -NR₂¹⁰, -SR¹⁰, -OSiR₃¹⁰, -SiR₃¹⁰ or -PR₂¹⁰ radical, in which R¹⁰ is a halogen atom, a C₁-C₁₀-alkyl group or a C₆-C₁₀-aryl group,
R⁴ to R⁶ are identical or different and have the meaning given for R³,
R⁷ is 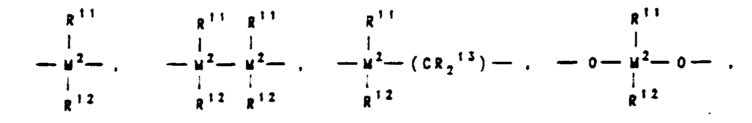 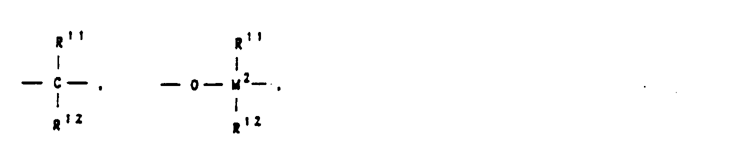
=BR¹¹, =AIR¹¹, -Ge-, -Sn-, -O-, -S-, =SO, =SO₂, =NR¹¹, =CO, = -PR¹¹ or P(O)R¹¹,
in which
R¹¹, R¹² and R¹³ are identical or different and are a hydrogen atom, a halogen atom, a C₁-C₁₀-alkyl group, a C₁-C₁₀-fluoroalkyl group, a C₆-C₁₀-aryl group, a C₆-C₁₀-fluoroaryl group, a C₁-C₁₀-alkoxy group, a C₂-C₁₀-alkenyl group, a C₇-C₄₀-arylalkyl group, a C₈-C₄₀-arylalkenyl group or a C₇-C₄₀-alkylaryl group, or R¹¹ and R¹², or R¹¹ and R¹³, in each case with the atoms joining them, form a ring,
aryl group, or R¹¹ and R¹², or R¹¹ and R¹³, in each case with the atoms joining them, form a ring,
M² is silicon, germanium or tin,
R⁸ and R⁹ are identical or different and have the meaning given for R¹¹ and
m and n are identical or different and are zero, 1 or 2, m plus n being zero, 1 or 2,
wherein R⁴ and R⁶ are not hydrogen.

10. A metallocene compound as claimed in claim 9, wherein, in formula I, R³ is hydrogen.

11. A metallocene compound as claimed in claim 9 or 10, wherein, in formula I, M¹ is Zr or Hf, R¹ and R² are identical or different and are methyl or chlorine, R⁴ and R⁶ are identical or different and are methyl, isopropyl, phenyl, ethyl or trifluoromethyl, R⁵ is hydrogen or has the meanings given for R⁴ and R⁶, R⁷ is a radical 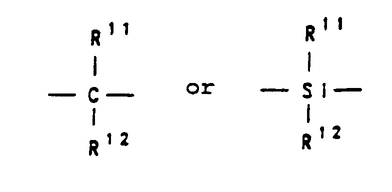 and m plus n is zero or 1.

12. A metallocene compound as claimed in claim 9, where the metallocene compound is rac-dimethylsilyl(2-methyl-4,6-diisopropyl-1-indenyl)₂zirconium dichloride, rac-dimethylsilyl(2,4,6-trimethyl-1-indenyl)₂zirconium dichloride, rac-phenyl (methyl)silyl(2-methyl-4,6-diisopropyl-1-indenyl)₂zirconium dichloride, rac-1,2-ethanediylbis(2-methyl-4,6-diisopropyl-1-indenyl)zirconium dichloride, or rac-1,2-butanediylbis(2-methyl-4,6-diisopropyl-1-indenyl)zirconium dichloride.

13. A catalyst component comprising a) a metallocene as claimed in one or more of claims 9-12 and b) a cocatalyst.

## Revendications

1. Procédé de préparation d'un polymère oléfinique par polymérisation ou copolymérisation d'une oléfine de formule R^{a}-CH=CH-R^{b}, dans laquelle R^{a} et R^{b} sont identiques ou différents et représentent un atome d'hydrogène ou un reste hydrocarboné de 1 à 14 atomes de carbone, ou bien R^{a} et R^{b} peuvent former un cycle avec les atomes qui les relient, à une température de -60 à 200°C, sous une pression de 0,5 à 100 bars, en solution, en suspension ou en phase gazeuse, en présence d'un catalyseur formé à partir d'un métallocène en tant que composé d'un métal de transition et d'un cocatalyseur, le métallocène étant un composé de formule I 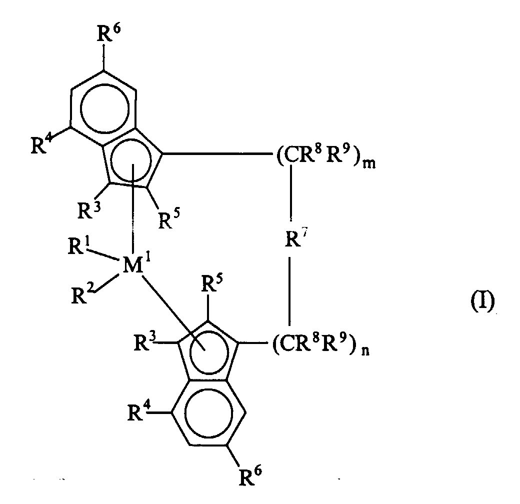 dans laquelle
M¹ est un métal du groupe IVb, Vb ou VIb du système périodique,
R¹ et R² sont identiques ou différents et représentent un atome d'hydrogène, un groupe alkyle en C₁-C₁₀, un groupe alcoxy en C₁-C₁₀, un groupe aryle en C₆-C₁₀, un groupe aryloxy en C₆-C₁₀, un groupe alcényle en C₂-C₁₀, un groupe arylalkyle en C₇-C₄₀, un groupe alkylaryle en C₇-C₄₀, un groupe arylalcényle en C₈-C₄₀ ou un atome d'halogène,
les restes R³ sont identiques ou différents et représentent un atome d'hydrogène, un atome d'halogène, un groupe alkyle en C₁-C₁₀ qui peut être halogéné, un groupe aryle en C₆-C₁₀ qui peut être halogéné, un reste -NR¹⁰₂, -SR¹⁰, -OSiR¹⁰₃, -SiR¹⁰₃, ou -PR¹⁰₂ dans lesquels R¹⁰ est un atome d'halogène, un groupe alkyle en C₁-C₁₀ ou un groupe aryle en C₆-C₁₀,
R⁴ à R⁶ sont identiques ou différents et ont les significations données pour R³,
R⁷ est 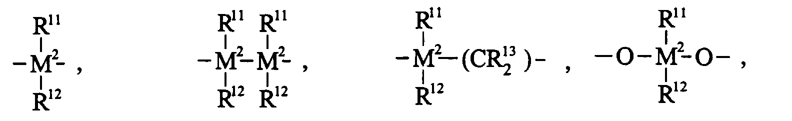 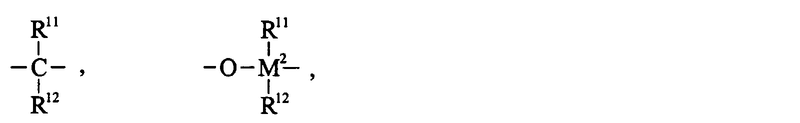
=BR¹¹, =AlR¹¹, -Ge-, -Sn-, -O-, -S-, =SO, =SO₂, =NR¹¹, =CO, =PR¹¹ ou =P(O)R¹¹,
où
R¹¹, R¹² et R¹³ sont identiques ou différents et représentent un atome d'hydrogène, un atome d'halogène, un groupe alkyle en C₁-C₁₀, un groupe fluoroalkyle en C₁-C₁₀, un groupe aryle en C₆-C₁₀, un groupe fluoroaryle en C₆-C₁₀, un groupe alcoxy en C₁-C₁₀, un groupe alcényle en C₂-C₁₀, un groupe arylalkyle en C₇-C₄₀, un groupe arylalcényle en C₈-C₄₀, un groupe alkylaryle en C₇-C₄₀, ou bien R¹¹ et R¹² ou R¹¹ et R¹³ forment respectivement un cycle avec les atomes qui les relient et
M² est le silicium, le germanium ou l'étain,
R⁸ et R⁹ sont identiques ou différents et ont la signification indiquée pour R¹¹, et
m et n sont identiques ou différents et sont égaux à 0, 1 ou 2, la somme m + n étant égale à 0, 1 ou 2,
caractérisé en ce que R⁴ et R⁶ ne représentent pas l'hydrogène.

2. Procédé selon la revendication 1, caractérisé en ce que, dans la formule I, R³ représente un atome d'hydrogène;

3. Procédé selon la revendication 1 ou 2, caractérisé en ce que, dans la formule I, M¹ est Zr ou Hf, R¹ et R² sont identiques ou différents et représentent un groupe méthyle ou un atome de chlore, R⁴ et R⁶ sont identiques ou différents et représentent un groupe méthyle, isopropyle, phényle, éthyle ou trifluorométhyle, R⁵ est un atome d'hydrogène ou a les significations indiquées pour R⁴ et R⁶, R⁷ est un reste 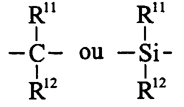 et la somme m + n est égale à 0 ou 1.

4. Procédé selon l'une ou plusieurs des revendications 1 à 3, caractérisé en ce que l'on utilise comme métallocène le dichlorure de rac-diméthylsilyl(2-méthyl-4,6-diisopropyl-1-indényl)₂zircomum.

5. Procédé selon l'une ou plusieurs des revendications 1 à 4, caractérisé en ce que l'on utilise comme cocatalyseur un aluminoxane de formule (II) 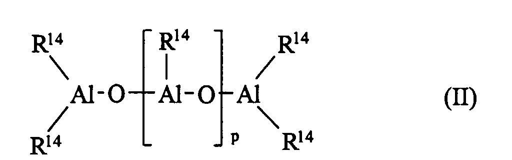 en ce qui concerne le type linéaire, et/ou de formule III 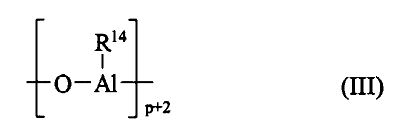 en ce qui concerne le type cyclique, où, dans les formules (II) et (III), les restes R¹⁴ sont identiques ou différents et représentent un groupe alkyle en C₁-C₆, un groupe aryle en C₆-C₁₈, un groupe benzyle ou un atome d'hydrogène et p est un nombre entier de 2 à 50.

6. Procédé selon l'une ou plusieurs des revendications 1 à 5, caractérisé en ce que l'on utilise un méthylaluminoxane comme cocatalyseur.

7. Procédé selon la revendication 5 ou 6, caractérisé en ce que l'on active au préalable le métallocène de formule I avec un aluminoxane de formule II et/ou III avant de l'introduire dans la réaction de polymérisation.

8. Utilisation d'un métallocène de formule I selon l'une ou plusieurs des revendications 1 à 4 comme catalyseur pour la polymérisation des oléfines.

9. Composé de métallocène de formule I 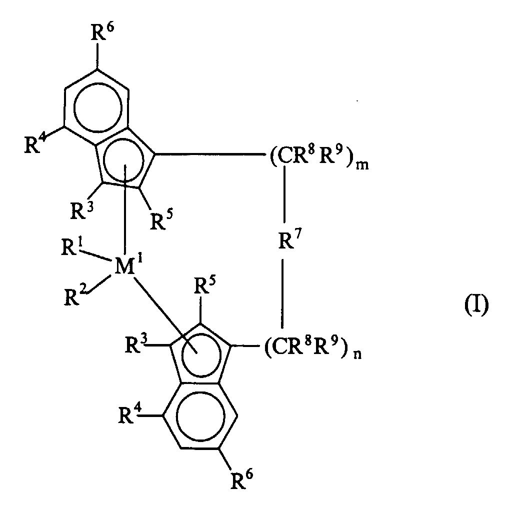 dans laquelle
M¹ est un métal du groupe IVb, Vb ou VIb du système périodique,
R¹ et R² sont identiques ou différents et représentent un atome d'hydrogène, un groupe alkyle en C₁-C₁₀, un groupe alcoxy en C₁-C₁₀, un groupe aryle en C₆-C₁₀, un groupe aryloxy en C₆-C₁₀, un groupe alcényle en C₂-C₁₀, un groupe arylalkyle en C₇-C₄₀, un groupe alkylaryle en C₇-C₄₀, un groupe arylalcényle en C₈-C₄₀ ou un atome d'halogène,
les restes R³ sont identiques ou différents et représentent un atome d'hydrogène, un atome d'halogène, un groupe alkyle en C₁-C₁₀ qui peut être halogéné, un groupe aryle en C₆-C₁₀ qui peut être halogéné, un reste -NR¹⁰₂, -SR¹⁰, -OSiR¹⁰₃, -SiR¹⁰₃ ou -PR¹⁰₂ dans lesquels R¹⁰ est un atome d'halogène, un groupe alkyle en C₁-C₁₀ ou un groupe aryle en C₆-C₁₀,
R⁴ à R⁶ sont identiques ou différents et ont les significations données pour R³,
R⁷ est 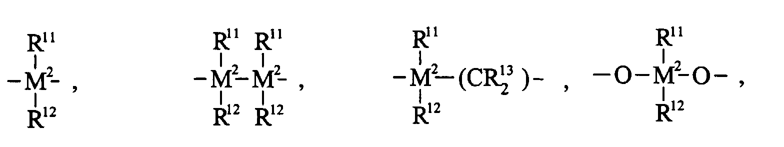 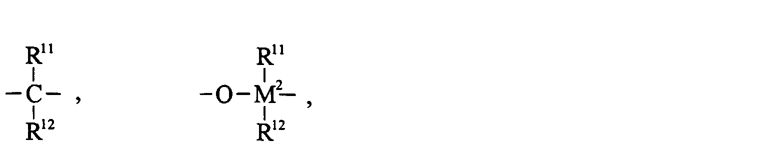 =BR¹¹, =AlR¹¹, -Ge-, -Sn-, -O-, -S-, =SO, =SO₂, =NR¹¹, =CO, =PR¹¹ ou =P(O)R¹¹,
où
R¹¹, R¹² et R¹³ sont identiques ou différents et représentent un atome d'hydrogène, un atome d'halogène, un groupe alkyle en C₁-C₁₀, un groupe fluoroalkyle en C₁-C₁₀, un groupe aryle en C₆-C₁₀, un groupe fluoroaryle en C₆-C₁₀, un groupe alcoxy en C₁-C₁₀, un groupe alcényle en C₂-C₁₀, un groupe arylalkyle en C₇-C₄₀, un groupe arylalcényle en C₈-C₄₀, un groupe alkylaryle en C₇-C₄₀, ou bien R¹¹ et R¹² ou R¹¹ et R¹³ forment respectivement un cycle avec les atomes qui les relient et
M² est le silicium, le germanium ou l'étain,
R⁸ et R⁹ sont identiques ou différents et ont la signification indiquée pour R¹¹, et
m et n sont identiques ou différents et sont égaux à 0, 1 ou 2, la somme m + n étant égale à 0, 1 ou 2,
caractérisé en ce que R⁴ et R⁶ ne représentent pas l'hydrogène.

10. Composé de métallocène selon la revendication 9, caractérisé en ce que, dans la formule I, R³ représente un atome d'hydrogène.

11. Composé de métallocène selon la revendication 9 ou 10, caractérisé en ce que, dans la formule I, M¹ est Zr ou Hf, R¹ et R² sont identiques ou différents et représentent un groupe méthyle ou un atome de chlore, R⁴ et R⁶ sont identiques ou différents et représentent un groupe méthyle, isopropyle, phényle, éthyle ou trifluorométhyle, R⁵ est un atome d'hydrogène ou a les significations indiquées pour R⁴ et R⁶, R⁷ est un reste 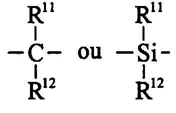 et la somme m + n est égale à 0 ou 1.

12. Composé de métallocène selon la revendication 9, le composé de métallocène étant:
le dichlorure de rac-diméthylsilyl(2-méthyl-4,6-diisopropyl-1-indényl)₂zirconium,
le dichlorure de rac-diméthylsilyl(2,4,6-triméthyl-1-indényl)₂zirconium,
le dichlorure de rac-phényl(méthyl)silyl(2-méthyl-4,6-diisopropyl-1-indényl)₂-zirconium,
le dichlorure de rac-1,2-éthanediylbis(2-méthyl-4,6-diisopropyl-1-indényl)zirconium ou
le dichlorure de rac-1,2-butanediylbis(2-méthyl-4,6-diisopropyl-1-indényl)zirconium,

13. Constituant de catalyseur contenant a) un métallocène selon l'une ou plusieurs des revendications 9 à 12 et b) un cocatalyseur.
